# EUROPEAN PATENT SPECIFICATION

(11) **EP 4 467 190 B1**
(45) Date of publication and mention of the grant of the patent: **25.11.2026**
(21) Application number: 23174965.6
(22) Date of filing: 23.05.2023
(51) Int. Cl.: A61N 5/06, A61N 5/067

(54) **APPARATUS AND NON-THERAPEUTIC METHOD FOR PATTERNED LASER TREATMENT OF A TISSUE SURFACE**
VORRICHTUNG UND NICHTTHERAPEUTISCHES VERFAHREN ZUR STRUKTURIERTEN LASERBEHANDLUNG EINER GEWEBEOBERFLÄCHE
APPAREIL ET PROCÉDÉ NON THÉRAPEUTIQUE DE TRAITEMENT LASER À MOTIFS D'UNE SURFACE DE TISSU

(43) Date of publication of application: 27.11.2024
(73) Proprietor: Fotona d.o.o., 1000 Ljubljana (SI)
(72) Inventor: LUKAC, Matjaz, 1000 Ljubljana (SI); KAZIC, Marko, 1233 Dob pri Domzalah (SI); KOSIR, Jure, 1230 Domzale (SI); CENCIC, Boris, 1000 Ljubljana (SI)
(74) Representative: Bardehle Pagenberg Partnerschaft mbB Patentanwälte Rechtsanwälte

(56) References cited:
- US-A1- 2008 021 442
- US-A1- 2013 178 917
- US-A1- 2018 221 199
- US-A1- 2022 205 611
- US-B1- 6 682 524

## Description

### 1. Field of the invention

The present invention relates to, inter alia, apparatuses for laser treatment of a tissue surface, non-therapeutic methods for shaping an illumination pattern of laser pulses for tissue treatment and respective computer programs.

### 2. Technical background

Non-ablative resurfacing of the skin and mucosa has in recent years attracted significant attention due to the technique's safety and excellent clinical results. Laser treatments with a laser comprising yttrium aluminum garnet doped with erbium ions (Er:YAG) have been determined to initiate changes in the cutaneous and mucosal tissues without causing unwanted direct epithelial ablation, if applied correctly. Through activation of the tissue healing response, fibroblasts are activated to migrate to the treated tissue volume, where they initiate the production of extracellular matrix and tissue remodeling. The non-ablative application of Er:YAG lasers has been, for example, very successfully used for face-lifting and skin tightening.

Smooth, non-ablative resurfacing comprises of delivering a sequence of laser pulses to the treated soft tissue, wherein a controlled number of laser pulses is delivered, with cumulative fluences (Fₛ) below the ablation threshold. During earlier, more aggressive non-ablative treatments, cumulative fluences close to or slightly above the ablation threshold were used. On the other hand, with smooth resurfacing, one tries to keep the Fₛ not only below the ablation threshold, but preferably also below a heat pain tolerance threshold.

Patients and physicians look for less invasive techniques to prevent or reverse signs of aging. For many years, ablative lasers have been used successfully for the treatment of wrinkles, but their use is limited by the associated pain and down time, and by the relatively high risk of side effects and complications. Therefore, the demand for non-ablative techniques that are minimally invasive yet effective is growing fast.

Many conventional approaches for tissue regeneration focus on a slow thermal pulsing of the connective tissue, in order to stimulate fibroblasts and other cells to respond to wound healing scenarios. The operation of according apparatuses is therefore limited by the above considerations, effectively resulting in long down times during which no laser light is delivered to the treated surface but the surface may cool down to remain below the pain threshold and to avoid ablation and/or damages to the treated surface in general. Present solutions therefore bear an increased danger of unwantedly heating at least some tissue layers above a tissue-specific threshold causing (possibly irreparable) damage therein.

US 2013/0178917 A1 relates to methods and systems for treatment, such as skin rejuvenation treatment, that use non-uniform laser radiation. A high-intensity portion of the laser radiation causes collagen destruction and shrinkage within select portions of the treatment area, while a lower-intensity portion of the radiation causes fibroblast stimulation leading to collagen production across other portions of the treatment area. An output beam from a laser source, such as an Nd:NAG laser, is coupled into an optical system that modifies the beam to provide a large-diameter beam having a nonuniform energy profile, comprised of a plurality of high-intensity zones surrounded by lower-intensity zones within the treatment beam. The higher-intensity zones heat select portions of the target tissue to temperatures sufficient for a first treatment (e.g. collagen shrinkage), while the lower-intensity zones provide sufficient energy for a second treatment (e.g. stimulated collagen production).

US 2008/0021442 A1 relates to systems and methods for performing fractional resurfacing of a target area of skin using electromagnetic radiation. An electromagnetic radiation is generated by an electromagnetic radiation source. The electromagnetic radiation is caused to be applied to a particular portion of a target area of skin. The electromagnetic radiation can be impeded from affecting another portion of the target area of the skin by a mask. Alternatively, the electromagnetic radiation may be applied to portions of the target area of the skin, other than the particular portion.

US 2018/0221199 A1 relates to an apparatus for treating macular degeneration including an optical filter configured to be mated with the ophthalmic lens, the filter having a surface wherein at least a portion of the surface comprises non-transmissive material that may distribute power from a light beam creating a plurality of light beams and a method of treating macular degeneration using the optical filter.

US 6,682,524 B1 relates to a dermatological handpiece by which a laser beam is directed to the surface of a selected skin area for purposes of a cosmetic treatment and the skin area is subjected to the action of the laser beam and a method for the cosmetic treatment of skin surfaces during the operation of this handpiece. A handpiece of the type described above is outfitted with a device for moderating the temperature of the skin surfaces on which the laser beam acted immediately beforehand and/or is to be subjected to the action of the laser beam immediately following the temperature moderation. The handpiece is further provided with elements for influencing the laser beam cross section with respect to shape, dimensioning and intensity distribution.

US 2022/0205611 A1 relates to an illumination apparatus for producing structured light and flood illumination, the illumination apparatus comprising a microlens array comprising microlenses which are arranged at a pitch P in at least a first direction, and a first array of first light sources and a second array of second light sources, the first light sources being configured to emit light at a wavelength L, wherein the first light sources are located at a distance D from the microlens array, wherein P2=2LD/N and N is an integer, and wherein a size of the second light sources is greater than a size of the first light sources, such that the light sources of the first array produce structured light and the light sources of the second array produce a continuous area of light.

There is therefore still a need to further improve (apparatuses for) laser treatment of a tissue surface, methods for shaping an illumination pattern of laser pulses for tissue treatment and respective computer programs and related aspects.

### 3. Summary

The invention is set out in the appended set of claims. Methods mentioned hereinafter concerning therapeutic treatments, such as treating scars, acne, age spots, sun damage, collagen shrinkage and promoting wound healing, do not form part of the present invention. Only non-therapeutic methods, such as hair removal or promoting production of collagen for skin rejuvenation, form part of the invention.

A first aspect of the invention relates to an apparatus for laser treatment of a tissue surface, the apparatus comprising at least one optical element for illuminating the tissue surface with at least one laser pulse having an illumination pattern, wherein the at least one optical element is configured such that the illumination pattern comprises at least one pattern element with a first width of less than 300 micrometers and the effective surface coverage of the illumination pattern more than 10%, wherein the illumination pattern comprises a mesh comprising the at least one pattern element and wherein the mesh comprises a honeycomb lattice.

This apparatus is based on an in-depth understanding of the functional crosstalk between skin layers and the heat transfer mechanisms associated with laser treatment of the skin. Essentially, the small first width allows for efficient and/or fast transversal cooling by transferring heat delivered by the at least one laser pulse to unheated tissue regions that were not illuminated. This allows in turn for higher laser pulse energy densities. This may especially induce temporally short intense heat spikes within the treated tissue. When the effective surface coverage amounts to 10% or more, more tissue may be treated simultaneously. The invention provides an advantageous way to allow for efficient cooling by forming illumination patterns as descried herein while illuminating the tissue surface with a high surface coverage. Thereby, the short first width, the illumination pattern, and the surface coverage as described herein result in a synergistic combination that improves the efficiency, safety, and operation speed of the apparatus for treating tissue surfaces.

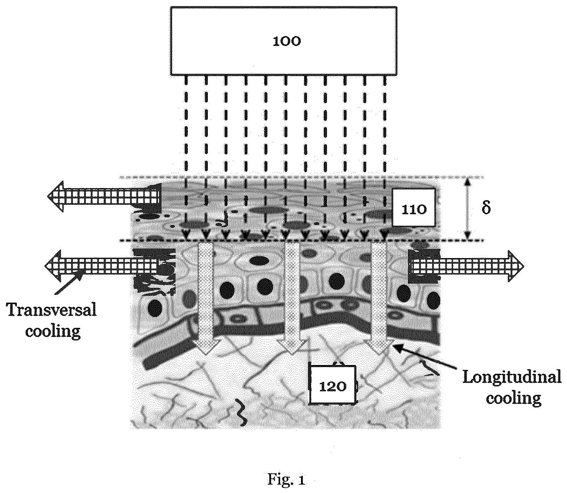

Fig. 1 illustrates heat transfer mechanisms, including longitudinal and transversal cooling, in an exemplary sample, namely skin, upon such laser treatment. In detail, the laser treatment in this example is delivered by the apparatus 100 illuminating the surface of the skin, the epidermis 110, where keratinocytes and other cells are located. In this superficial layer above the basement membrane, the keratinocytes and other cells are activated (i.e., triggered) by a fast and/or intense heat shock induced by thermal pulses delivered by the device 100.

During and immediately following the delivery of the individual laser pulses, e.g., as described herein, rapid longitudinal cooling deeper into the tissue occurs that dominates the cooling dynamics. However, during the time in-between laser pulses, the slower transverse cooling due to the heat diffusion in the radial direction away from a laser irradiated spot may become important as well. Altogether, both heat transfer mechanisms may occur on different timescales and cooperatively redistribute the energy or heat delivered by the laser treatment. Thereby, not only the volume down to a depth δ that is penetrated by the laser may be affected by the laser treatment but also the volume 120 that the excess heat induced by the laser treatment is transferred to, i.e., especially the fibroblasts.

A fibroblast is a type of biological cell that synthesizes the extracellular matrix and collagen, produces the structural framework (stroma) for animal tissues, and plays a critical role, e.g., in wound healing. The fibroblast 120 is the site-specific cell of connective tissue that plays an important role in the formation and degradation of the intercellular substance, the extracellular matrix. Keratinocytes 110 are the primary type of cell found in the epidermis, the outermost layer of the skin. In humans, they constitute 90% of epidermal skin cells. Among other functions, keratinocytes 110 form a barrier against environmental damage by UV radiation, i.e., they absorb incoming light. In healthy skin, keratinocytes 110 participate to maintain skin homeostasis by actively cross-talking with fibroblasts 120.

The present invention allows exploiting just this crosstalk: New collagen production may be triggered not by the direct temperature elevation of the connective tissue but instead by the cross talk between the deeper lying fibroblasts and the superficially located keratinocytes that are activated by the fast thermal pulse triggering delivered by the apparatuses and methods described herein: In detail, the rapidly heated thin superficial tissue layer exposed to the laser treatment is quickly cooled down by the fast diffusion of the generated heat to the underlying colder tissue layers. This is caused by the temperature gradient, which is very large in the superficial thin layer. As a result, the conductive cooling of the superficial layer is very effective, in contrast to within deeper connective tissue layers that remain moderately heated for a significantly longer time, owing to weaker heat flow from this region. Since the protein denaturation rate depends on temperature in a highly nonlinear manner, the short-lived high temperature gradient can be used to trigger deeper tissue regeneration without thermally damaging the epithelial tissue.

In light of the above context, the apparatus according to the first aspect allows for efficient and/or fast transverse cooling as the at least one pattern element comprises a width of only 300 micrometers or less. Therefore, as opposed to connective tissue heating where heat diffusion prolongs exposure times, it is the fast thermal diffusion from the heated thin superficial tissue layer that facilitates the generation of extremely short thermal exposure pulses. Additionally, due to the fast longitudinal thermal diffusion from the heated thin superficial tissue layer (ca. 1 - 3 µm with Er:YAG laser), the duration of the thermal exposure (tₑ) to high temperature peaks may be extremely short (< 3.5 ms with Er:YAG laser). This may beneficially reduce down-times required to prevent potential tissue damage as described herein. At the same time, the patterning may allow to essentially treat a larger area simultaneously and/or evenly, reducing the time required for respective procedures.

The laser treatment of the tissue surface may, e.g., be a regenerative skin laser treatment and relate to a non-invasive cosmetic procedure that uses laser technology, e.g., to stimulate the growth and/or renewal of skin cells. The treatment may comprise directing a focused beam of light onto the surface of the skin, which penetrates the outer layers of the skin to reach deeper tissue layers. During the treatment, the laser energy may trigger a natural healing response in the skin, which prompts the production of new collagen and elastin fibers. These fibers help to improve the texture and tone of the skin, reduce the appearance of fine lines and wrinkles, and promote overall skin rejuvenation. Regenerative skin laser treatment may be used to address a range of skin concerns, including, e.g., sun damage, age spots, acne scars, and uneven skin tone.

The apparatus may further comprise one or more means for generating laser pulses, such as laser sources, e.g., connected in series or in parallel. The at least one optical element may comprise one or more light shaping elements. Light shaping elements may be, e.g., optical components designed to control the direction, intensity, and/or distribution of light. Some examples for light shaping elements may comprise lenses, micro-lenses, mirrors, diffusers, reflectors, prisms, gratings, and/or combinations thereof. The apparatus may, e.g., be a handheld apparatus, a so-called handpiece. Alternatively, the components of the apparatus may be distributed over a stationary portion and a handpiece. In such examples, a laser source may, e.g., be comprised in the stationary part and the optical element for determining the illumination pattern may be comprised in the handpiece, wherein, e.g., the laser source may be connected with the handpiece by a flexible optical fiber or articulated arm and/or feed the handpiece with laser pulses. The handpiece, in any example, may then easily positioned relative to the tissue surface to be treated.

In an example, the apparatus and/or the optical element may further comprise a laser source, preferably an infrared laser source, more preferably an Er:YAG laser source.

Laser sources may advantageously be configured to provide sufficiently high energy densities, pulsed illumination and especially Er:YAG proved to be suitable for the treatment of tissue surfaces.

Infrared lasers may, e.g., comprise devices that emit laser light in the infrared region of the electromagnetic spectrum, e.g., with a wavelength of 750 nm to 12000 nm. Such infrared lasers may be continuous wave lasers, e.g., to be used as pump lasers, or pulsed lasers. In more detail, an infrared laser may comprise a laser cavity that contains an active medium, such as a crystal, semiconductor or gas, and an optical or other type of pumping system that provides energy to the active medium to produce a population inversion. The laser cavity may also comprise a resonant structure, such as mirrors or gratings, that reflects and amplifies the light produced by the active medium.

Examples of infrared lasers may comprise, e.g., the following: CO₂ lasers use a mixture of carbon dioxide, nitrogen, and helium gases to produce laser light with a wavelength of 9.3-10.6 µm. Nd:YAG lasers may use a YAG crystal doped with neodymium ions to produce laser light with a wavelength of 1.064 µm. Quantum cascade lasers may use quantum mechanics to produce laser light in the mid-infrared region of the spectrum, typically between 4 and 12 µm. Semiconductor lasers may use semiconductor materials, such as gallium arsenide or indium phosphide, to produce laser light with a wide range of wavelengths, including the infrared region. Er:YAG lasers may use a crystal of yttrium aluminum garnet (YAG) or alternatively, an yttrium-scandium-gallium garnet (YSGG) doped with erbium ions may be used to produce laser light with a wavelength of 2.94 µm or 2.79 µm, respectively. It may be preferred for use in dental procedures, skin resurfacing, and other medical applications.

In one example, the apparatus may, e.g., be an apparatus for non-ablative tissue treatment and/or regeneration. The apparatus may, e.g., comprise an Er:YAG laser for generating the at least one laser pulse comprising a wavelength and/or means for directing the at least one laser pulse onto the tissue surface, e.g., of a human or animal body. The at least one optical element comprising, e.g., a laser source, may be adapted so that an energy delivery time t_{ed} of the at least one laser pulse, during which the second half of the pulse energy may be delivered, may be sufficiently short so that, given the wavelength and thus a corresponding penetration depth δ (see, e.g., Fig. 1) of the at least one laser pulse, the thermal exposure time is less than 2 ms, 1.5 ms, 1.0 ms, 1 ms, 900 µs, 600 µs or 300 µs. The thermal exposure time may be approximated as t_{ed} + (1/D)(δ+(2Dt_{ed})^{0·5})² (e.g., with a thermal diffusivity D of typical soft tissue as an example for tissue surfaces to be treated by the apparatus of D = 0.1 mm²s⁻¹). Preferably, the at least one optical element of the apparatus may be adapted to determine the wavelength of the at least one laser pulse so that the penetration depth δ of the at least one laser pulse is smaller than 30 µm, 10 µm, or 4 µm. The wavelength of the at least one laser pulse may be determined to be between 2.6 µm and 3.2 µm or between 9.1 µm and 11.2 µm, in order to coincide with the mid-infrared water absorption peaks of water which is the major constituent of human and body tissues.

Additionally or alternatively, the pulse energy of the at least one laser pulse may be chosen such that the fluence on the tissue surface may be below 1.5×10⁴ J/cm³xδ, wherein 1.5×10⁴ J/cm³ is the specific heat of ablation for soft tissue. Notably, the fluence may thus depend on the penetration depth δ. As an example, the fluence defining the ablation threshold may, e.g., be 1.5 J/cm² for an Er:YAG laser. The fluence may therefore be, e.g. for an Er:YAG laser, chosen to be in the range from 0.2 to 0.4 J/cm². The pulses described herein may generally comprise properties, in particular, temporal properties as described in EP 3 569 287 A1 which is incorporated by reference in full.

Generally, the illumination pattern may relate to a series of repeating pattern elements that are arranged in a regular manner. The pattern elements may, e.g., comprise geometrical shapes like lines, circles, triangles, squares, rhomboids, and/or hexagons. The pattern elements may, e.g., comprise an areal illumination in the shape of the respective pattern element, and/or an illumination in form of the contours of the pattern elements. In some examples, wherein the pattern element comprises the contours of a geometrical element, the first width may, e.g., be defined by the width of the contour of the pattern element. In examples, wherein the pattern element comprises an areal illumination in form of a geometrical element, the first width may, e.g., be defined by the width of the pattern element along one axis, e.g., along the direction corresponding to the smallest width.

The pattern elements may be arranged in various configurations and orientations, e.g., in a connected or disconnected way, to create the illumination pattern. A pattern may comprise a repetition of only one congruent pattern element and/or different pattern elements. The illumination pattern may in form boundary lines of said pattern elements.

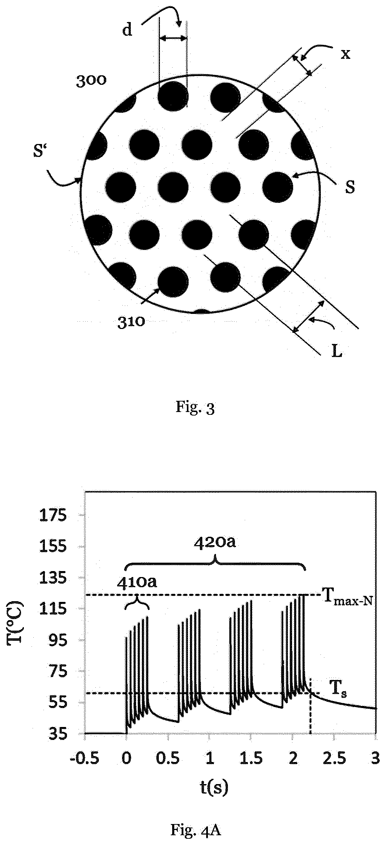

The effective surface coverage T_{C} of the illumination pattern may in other embodiments also be equal to 10% or less than 10%. In an exemplary embodiment, the surface coverage T_{C} may also be, e.g., above 15%, 20%, or 25%. Keeping the effective surface T_{C} above a certain threshold may result in sufficient total amounts of energy to be deposited into the tissue surface to be treated. When T_{C} is too low, the over-all efficiency of the apparatus may be impaired such that the time required to perform the treatment may increase. The covered surface area is understood herein as determined by the area within the full width half maximum (FWHM) in all directions covered by the pattern elements of the illumination pattern. The surface coverage is defined by the covered surface area divided by a well-defined total area that comprises the whole illumination pattern, i.e. the smallest circle around the illumination pattern (see, e.g., Fig. 3), or the smallest polygon that can be fitted around the entire illumination pattern in case of a non-circular pattern. In examples, where the illumination pattern comprises a lattice, as described herein, the surface coverage may be defined by the area of all pattern elements within one unit cell divided by the area of the unit cell.

The pattern element may, e.g., be any geometrical element, e.g., one or more rounded elements, elements comprising corners, straight and/or curved lines, etc. The pattern elements may, e.g., be blurred at their edges. Their first width may therefore be defined by their FWHM. The FWHM may be applied as a suitable measure for the width also to pattern elements with non-Gaussian intensity profiles. In one example, wherein the pattern element comprises a line, a first width perpendicular to the primary extension of the line may be less than 300 micrometers while a second width along the primary extension of the line may be more than 300 micrometers. In some examples, the pattern may comprise pattern elements with a first and a second width both below 300 micrometers. The width may be the width of said pattern element when the tissue surface is illuminated by the illumination pattern at a typical working distance of the apparatus. In some examples, the pulses may leave the at least one optical element in a collimated manner, such that the dimensions of the illumination pattern are essentially independent from the working distance. In some examples, the apparatus may be adapted to be in contact with the tissue surface, such that the correct working distance is automatically ensured.

Thereby, the apparatus described herein may differ from conventional approaches for tissue regeneration which focus on a slow thermal pulsing of the connective tissue to stimulate fibroblasts and other cells to respond to wound healing scenarios. In contrast, the approach according to the first aspect may base on an indirect mechanism whereby the keratinocytes and other cells located in the superficial layer above the basement membrane are activated (i.e., triggered) by extremely fast and intense "heat shock" thermal pulses. The new collagen production is thus triggered not by the direct temperature elevation of the connective tissue but instead by the cross talk between the deeper lying fibroblasts and the superficially located keratinocytes that are activated by the fast thermal pulse triggering delivered by our innovative apparatus and method.

Overall, regenerative skin laser treatment provides a safe and effective option for individuals seeking to improve the appearance and health of their skin without undergoing invasive surgical procedures and provides an over-all beneficial improvement:
The rapidly heated thin superficial tissue layer following a laser pulse is quickly cooled down by the fast diffusion of the generated heat to the underlying colder tissue layers. This is caused by the temperature gradient, which is very large in the superficial thin layer. As a result, the conductive cooling of the superficial layer is very effective, in contrast to within deeper connective tissue layers that remain moderately heated for a significantly longer time, owing to weaker heat flow from this region. Since the protein denaturation rate depends on temperature in a highly nonlinear manner, the short-lived high temperature gradient can be used to trigger deeper tissue regeneration without thermally damaging the epithelial tissue. Altogether, this may yield various advantages including, e.g., improved/faster cooling dynamics, less damage risk and/or less time required for according laser treatment procedures.

In an example, a pulse energy of the at least one laser pulse may be within a range such that the corresponding fluence on the tissue surface corresponds to a non-ablative treatment of the tissue surface.

This may yield the following advantageous technical effects: Non-ablative treatments may be associated with fewer complications such as scarring, infection, and changes in skin pigmentation than ablative treatments. Non-ablative treatments may be less likely to cause side effects such as redness, swelling, and pain, compared to ablative treatments. Importantly, non-ablative treatments may be done more frequently than ablative treatments. This may especially be beneficial when multiple sessions may be necessary to achieve the desired results. This allows for a gradual improvement, e.g., in skin tone and/or texture, without the need for a single, more aggressive treatment. Non-ablative treatments may be typically safe and effective for a wider range of skin types, including darker skin tones that may be more prone to complications with ablative treatments. Overall, non-ablative treatments may provide a safer and more effective way to treat tissue surfaces without the risks associated with, e.g., more invasive ablative treatments.

Non-ablative treatment of tissue surfaces may relate to medical procedures that aim to improve the appearance or function of tissue without removing or damaging the tissue surface. Such procedures may use lasers to penetrate the tissue without causing significant damage or removal of the outer layer. Non-ablative treatment can be used for a variety of applications, such as skin rejuvenation, scar reduction, and hair removal. Therefore, the energy may be delivered at a controlled intensity and duration to avoid damage to the outer layer of the tissue.

To achieve effective non-ablative treatment of tissue surfaces, at least one of the following exemplary requirements may be met: The energy delivered to the tissue surface may be sufficient to achieve the desired biological response without causing significant damage or removal of the outer layer of tissue, the energy may be delivered in a controlled and precise manner to avoid unintended damage to surrounding tissue, the energy source used may be compatible with the tissue being treated and must be capable of delivering the desired energy parameters, and/or the treatment may be tailored to the specific needs of the patient, taking into account factors such as skin type, age, and medical history.

Overall, non-ablative treatment of tissue surfaces may offer a safe and effective alternative to traditional ablative treatments.

For example, the at least one pulse may have a pulse length shorter than 2 ms, 1.5 ms, 1.0 ms, 1 ms, 900 µs, 600 µs or 300 µs. Additionally or alternatively, the thermal exposure time, as described herein, may be less than 2 ms, 1.5 ms, 1.0 ms, 1 ms, 900 µs, 600 µs or 300 µs.

Since skin is known to be highly sensitive to temperature, treatments may be configured such that certain, potentially tissue-specific temperature thresholds are not exceeded during the treatment of the tissue surface by the apparatus described herein. However, surprisingly, it was discovered that no irreversible thermal injury is expected for temperatures up to and even above 250 °C provided that the thermal exposure time is shorter than approximately 1 - 2 ms. Therefore, reducing the thermal exposure time allows for significantly more efficient treatment of the tissue surface achieving high temperatures with a low risk for causing tissue damage. In detail, it was found that a critical temperature T_{crit} at which tissue is damaged strongly depends on how long the tissue is exposed (tₑ) to temperatures of or above said T_{crit}: For example, for tₑ ≈ 200 s, T_{crit} ≈ 50 °C, for tₑ ≈ 0.1 s, T_{crit} ≈ 70 °C, and for tₑ ≈ 900 µs, T_{crit} ≈ 250 °C. Notably T_{crit}(tₑ) exhibits a sudden steep increase for decreasing tₑ at below ca. 0.01 s.

Considering the above, typical skin resurfacing lasers such as Er:YAG or CO₂ lasers, the thermal exposure time may be tₑ ≈ 0.4 ms for the Er:YAG laser (for λ = 2,940 nm, δ ≈ 1 µm) and tₑ ≈ 3.9 ms for the CO₂ laser (λ = 10640 nm, δ ≈ 15 µm). These numbers assume that the energy delivery times for both lasers of t_{ed} = 0.1 ms. This translates to T_{crit} > 300 °C for the Er:YAG laser and T_{crit} = 110 °C for the CO₂ laser. Similarly, the Er,Cr:YAG laser (λ = 2,780 nm, δ ≈ 3 µm) with t_{ed} = 0.1 ms, may have an exposure time of tₑ ≈ 0.7 ms which translates into T_{crit} ≈ 250 °C. It follows that, for this energy delivery time, only the Erbium lasers have a critical temperature above or at the boiling temperature of about T_{b} = 250 °C of typical/exemplary human and/or animal tissue.

The optical element may provide laser pulse sequences comprising at least one laser pulse through various methods, e.g., by one of the following: Mode-locking may involve using a device within the laser cavity, such as a saturable absorber, to create a series of ultra-short pulses that are spaced evenly apart. The result may be a train of laser pulses with high peak powers and short pulse durations. Q-switching may rely on using a device, such as an acousto-optic or electro-optic modulator, to create a high-Q resonant cavity that can store energy for a short period of time. When the energy in the cavity reaches a threshold level, it may be released in a single pulse, resulting in a high-energy, short-duration laser pulse. Chirped pulse amplification (CPA) may involve stretching a short-duration laser pulse in time using a device such as a diffraction grating or prism. The pulse may then be amplified to high energies before being compressed back to its original duration. This method allows for the generation of extremely high-energy laser pulses with durations as short as a few femtoseconds. Burst mode or free generation methods may involve rapidly switching the laser on and off in a controlled manner to produce a series of laser pulses with a variable time delay between them. Frequency modulation may involve modulating the frequency of the laser output to produce a sequence of pulses with a variable time delay between them. Overall, these methods allow for the generation of a wide variety of laser pulse sequences that can be tailored to the requirements of the tissue to be treated.

For example, the tissue may be predetermined, and the apparatus may be configured to deliver the at least one laser pulse, such that each of the at least one laser pulse may heat the tissue surface to a temperature above 70°C.

Reaching said minimal temperature may improve the efficiency of the apparatus and the treatment it may deliver to the tissue surface as the response of the tissue may rely strongly on the temperatures induced in the tissue by the at least one laser pulse.

The temperature of a tissue surface irradiated by a laser pulse sequence comprising the at least one laser pulse may, e.g., be controlled by adjusting the laser parameters such as pulse duration, energy, pulse shape and/or repetition rate. In order to heat the tissue surface to a temperature, e.g., of 70°C, 80°C, 90°C, 100°C, 125°C, 150°C, 175°C, 200°C, 225°C or 250°C, by each of the at least one laser pulse, at least one laser parameter (e.g., pulse energy, illumination pattern measures, and/or pulse duration, etc.) may be carefully selected to ensure that sufficient energy is delivered to the tissue surface without causing thermal damage.

The amount of energy delivered by each laser pulse and/or the time interval between each pulse may be adjusted to control the temperature of the tissue surface. For example, when the laser pulse energy is increased, the tissue surface will absorb more energy, which will result in an increase in temperature. Similarly, when the time interval between each pulse is decreased, the temperature of the tissue surface will increase as a result of the cumulative effect of the laser pulses. To ensure that the tissue surface temperature exceeds a certain minimum temperature, e.g., 70°C, during the illumination by one laser pulse, it is important to select laser parameters that deliver sufficient energy to the tissue surface. The minimum temperature that needs to be achieved may depend on the specific tissue and the desired biological response.

In an example, the apparatus may further comprise means for receiving a user input regarding the tissue surface to be treated and means for automatically adapting at least one parameter of the at least one laser pulse based at least partly on the user input.

Adjusting at least one parameter of the laser pulse such that it is adapted to the user input and thereby to the specific tissue it is directed to may improve the efficiency and reliability of the apparatus and may reduce the risk of inefficient treatment with too low pulse energies and/or causing damage with too high pulse energies.

There may be several exemplary types of user input that may be used to provide information on the (e.g., human or animal) tissue surface, including for example: a tissue type (skin, mucosa, nails, etc.), a location on a body (e.g., face, arm, leg, etc.), a patient age, appearance, color, texture, and other visual and/or haptic characteristics (e.g., obtained from direct or indirect visual examination of the tissue surface).

Additionally or alternatively, more profound user input may, e.g., comprise information gathered by examining tactile feedback (e.g., the use of touch to gather information about the tissue surface, such as its texture, firmness, or elasticity), imaging techniques (e.g., such as ultrasound, magnetic resonance imaging, computed tomography, and/or optical coherence tomography may provide detailed information about the internal structure of the tissue surface), histological examination (e.g., the removal of a small sample of tissue for laboratory analysis may provide information about the tissue structure and composition), electrical impedance or capacitance measurement (e.g., the use of electrodes to measure the electrical impedance or capacitance of the tissue surface may provide information about its structure and composition), and/or temperature measurements (e.g., the use of temperature sensors to measure the temperature of the tissue surface, may provide information about its metabolic activity and other physiological processes). Overall, only one or a combination of at least two of these different types of user input may provide a comprehensive understanding of the type of human or animal tissue surface being treated.

For example, the illumination pattern may comprise a mesh comprising the at least one pattern element. Such mesh-shaped illumination may advantageously combine a suitable surface coverage of the illumination pattern such that both, transverse and longitudinal heat transfer may occur efficiently from which the over-all treatment may benefit as described herein.

An illumination pattern in the form of a mesh may refer to an illumination pattern of laser light that is arranged in a series of, e.g., intersecting lines and/or grids, creating a mesh-like appearance. The laser illumination pattern in the form of a mesh may be generated using a variety of techniques, such as, e.g., using a series of mirrors and/or diffraction gratings to split and redirect the laser beam. The mesh pattern may be customized to achieve different levels of complexity and density, allowing for a wide range of embodiments.

In some examples, the at least one pattern element may have a line shape and/or may form at least a part of a line shape. The pattern elements themselves may increase their perimeter-to-area ratio and thereby improve the efficiency of heat transfer from the area of the pattern element to the surrounding tissue. This may improve the apparatus and the over-all treatment as described herein, e.g., in terms of reduced down-times, reduced damage risk, etc.

For example, each of the lines of a mesh may comprise a pattern element as described herein. E.g., the at least one pattern element may be a line of a certain length and form the whole line of the mesh or a part thereof. Exemplary meshes may comprise multiple pattern elements, e.g., linear pattern elements, that may vary or be similar in terms of their shape, size, orientation, and/or intensity profile, etc. A plurality of pattern elements may be provided to the tissue surface at the same or at different times. For example, triangular, square, and/or hexagonal patterns as described herein may comprise pattern elements, e.g., lines, connecting nearest neighbor corner points of the associated triangles, squares, and/or hexagons. Thereby, the pattern elements may, e.g., have a (line) width of less than 300 micrometers.

In some examples, the first width may correspond to a width of the line shape. The line shape may have a length of at least 4 times, preferably at least 10 times of the first width. Such shapes may have a particularly beneficial perimeter-to-area ratio and yield the advantages described herein in this context, e.g., an accelerated heat-transfer to surrounding tissue compared to more compact/less elongated pattern elements like spot-shapes.

As described herein, the pattern elements do not necessarily have sharp edges, but their width and length may be defined as described herein, e.g., by the FWHM. The length of the pattern element may also be referred to as a second width and measured analogously. For example, when the length of the pattern element is at least 4 or 10 times the width of the pattern element, the pattern element may have a substantially linear shape. Note that the term "shape" refers to the shape of the illumination projected onto a tissue surface, which may not necessarily be a perfectly straight line due to factors such as surface roughness or curvature. Linear pattern elements may, e.g., be straight or curved by design and have a varying first width along their extension (i.e., length). In such examples, the first width may be a measure of the width at a given position essentially perpendicular to the extension of the pattern element at that position.

In some examples, the mesh may comprise a lattice, preferably a honeycomb lattice. Lattices as described herein comprise a regular arrangement which may result in the context of the invention in an essentially even illumination of the tissue surface to be treated. This may facilitate the use of the apparatus and/or make it more reliable and safer.

A two-dimensional lattice may be based on two sets of vectors: Firstly, the lattice vectors a₁ and a₂ define the periodicity of the lattice and determine the shape of the unit cell, which is the smallest repeating unit of the lattice. A unit cell is located at each point defined by the lattice vectors: m₁·a₁ + m₂·a₂ | mᵢ ∈ Z, where a₁ and a₂ are linearly independent vectors known as the lattice vectors of the lattice, Z is the set of integers, and m₁ and m₂ represent the coordinates of a point in the lattice. set of basis vectors, which define the relative positions of characteristic lattice points relative to the positions defined by the lattice vectors. Secondly, a lattice as described herein may comprise one or more basis vectors b₁, b₂, ... which may define where pattern points are located within each unit cell. The positions of the pattern points j are determined by m₁·a₁ + m₂·a₂ + bⱼ· Often, for simplicity, one basis vector may be chosen as (0, 0).

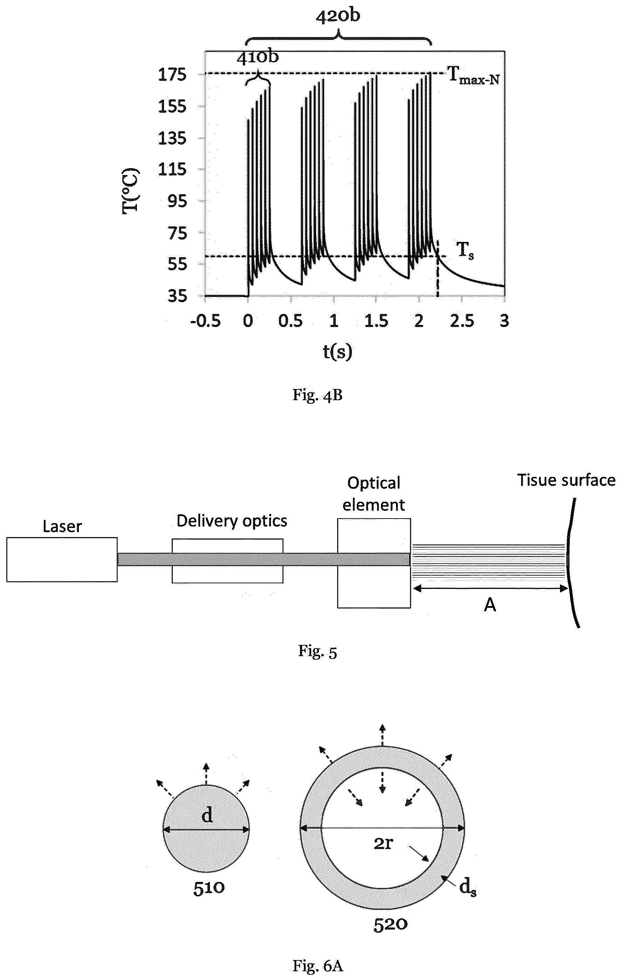

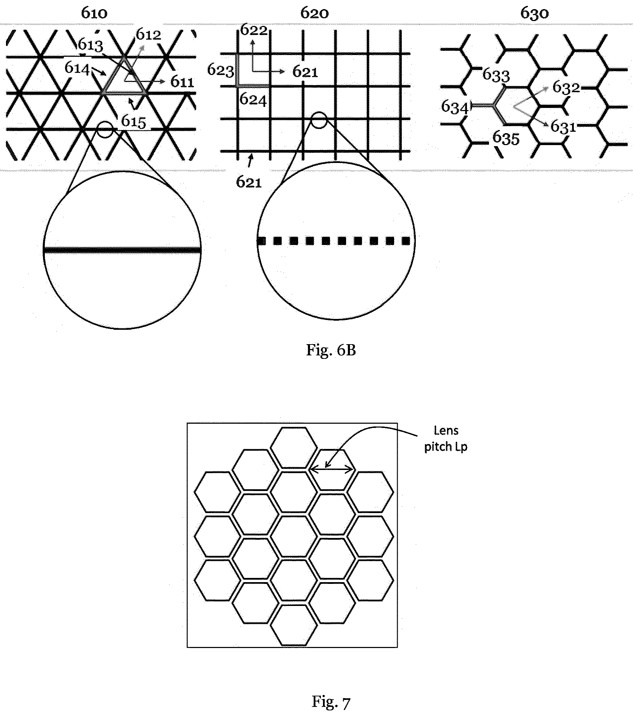

For example, for a triangular lattice, one may define a₁ = a (0, 1), a₂ = a/2 (3^{0.5}, 1), and b₁ = (0,0). For a square lattice, one may define a₁ = a (1, 0), a₂ = a (0, 1), and b₁ = (0,0). As in both examples for the triangular and the square lattice have only one basis vector b₁ = (0,0), the pattern points are located right at the lattice points m₁·a₁ + m₂·a₂. For a honeycomb lattice, one may define: a₁ = a/2 (3, 3^{0.5}), a₂ = a/2 (3, -3^{0.5}), b₁ = (0,0), and b₂ = (a,0) (see also Fig. 6). The honeycomb pattern/lattice requires two basis vectors b₁ and b₂, describing the position of two pattern points within the unit cell. The pattern points are therefore located at m₁·a₁ + m₂·a₂ (for b₁ = (0, 0)) and at m₁·a₁ + m₂·a₂ + b₂ (for b₂ = (a,0)).

A mesh as described herein may, e.g., be created by pattern elements, e.g., linear pattern elements, that are placed in a predetermined orientation relative to the characteristic points defined by the lattice and base vectors. For example, linear pattern elements may be placed between said characteristic points and/or connect said characteristic points such as to create a mesh of connected or unconnected pattern elements.

For example, the lattice may comprise at least one lattice vector with a length in a range from 0.1 mm to 10 mm, 0.5 mm to 5 mm, or 1 mm to 3 mm. These dimensions may be particularly suitable, considering typical heat transfer lengths in human and/or animal tissue to be treated. They may yield an ideal range wherein heat may be efficiently distributed and at the same time, patterns may be resolved sufficiently when illuminating the tissue surface avoiding potential disadvantages associated with full spot illumination as described herein.

In an example, the effective surface coverage T_{C} of the illumination pattern may be less than 50%, less than 40%, or less than 30%. When T_{C} is too high, the over-all efficiency of the apparatus may be impaired as there is no non-illuminated tissue that heat may be transferred to.

In some examples, the illumination pattern may form boundary lines of a tessellation pattern, preferably a regular tessellation pattern. In the context of the invention, tessellation patterns yield the advantages associated with a regular illumination of the tissue to be treated while simultaneously leaving gaps resulting in non-illuminated tissue that heat may be transferred to. Therefore, the use of illumination patterns in form of tessellation patterns may improve the efficiency, reliability, and over-all performance of the apparatus.

Tessellation patterns may comprise identical shapes arranged in a repeated manner to cover an area without any gaps or overlaps. The shapes comprised in tessellation patterns may be simple, such as squares, triangles, or hexagons, or more complex, such as irregular polygons or even curved shapes. They may regularly cover an area in whole. The shapes used in the pattern may be arranged in a way that fully fills the space without leaving any gaps or overlaps. This regularity is achieved by carefully selecting the shape and size of the tiles, as well as the way they are arranged. There are different types of tessellation patterns, including regular tessellations, which use only one type of regular polygon to fill the plane, and semi-regular tessellations, which use two or more types of regular polygons in a repeating pattern. There are also non-regular tessellations, which use non-regular polygons or curved shapes to fill the plane. Thereby, tessellation patterns combine to beneficial aspects namely a patterning of the illumination with a limited surface coverage and a regularity providing an essentially evenly distributed treatment of the tissue surface over the whole pattern.

In some examples, the at least one optical element may be switchable between at least a first and a second configuration. The at least one pattern element may comprise a first geometrical shape when the at least one optical element is in the first configuration and the at least one pattern element may comprise a second geometrical shape when the at least one optical element is in the second configuration, wherein the first geometrical shape may be different from the second geometrical shape.

For example, the illumination pattern and/or the at least one pattern element may be determined in terms of its geometrical shape relating to, e.g., its shape (e.g., circle, ring, and/or hexagon), size, orientation, and/or intensity (distribution). Switching between the first and second configuration may, e.g., comprise adjusting a distance between the at least one optical element and the tissue surface and/or (mutual) distances and/or (mutual) orientations between two or more components of the at least one optical element. Additionally or alternatively, e.g., when the optical element comprises a laser, switching between the first and second configuration may, e.g., comprise adjusting laser parameters such as, e.g., a pulse repetition rate, a laser wavelength, a laser fluence, a laser intensity, and/or a laser focus, etc. As a result of switching between the first and second configuration, the illumination pattern and/or the at least one pattern element may be varied in terms of its geometrical shape relating to, e.g., its shape (e.g., circle, ring, and/or hexagon), size, orientation, and/or intensity (distribution).

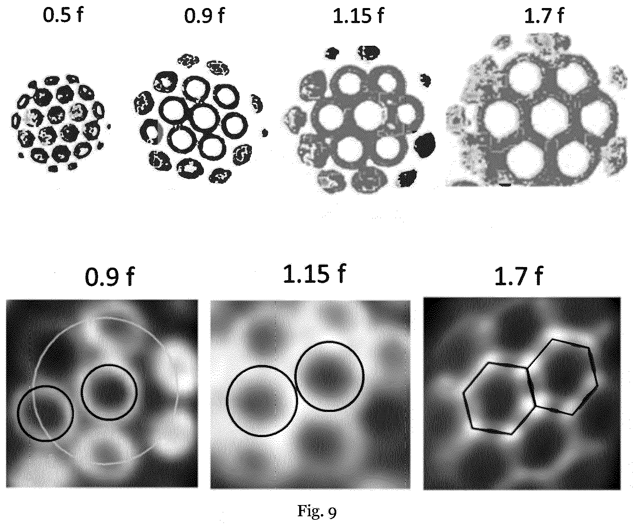

For example, an illustrative scenario may be considered, wherein the pulse repetition rate is 40 Hz and a beam quality ratio (M²), a laser divergence and a wavefront radius at the location of, e.g., a micro lens array comprised in the at least one optical element are the largest: At these conditions the honeycomb pattern may develop at a distance A between the at least one optical element and the tissue surface of 1.7 times the focal length f of the micro lens array. When the repetition rate is reduced, resulting in a lower laser beam divergence due to the lower thermal focusing within the laser rod, the distance A where the hexagonal pattern develops becomes larger/longer. Therefore, by adjusting the laser beam characteristics by increasing or decreasing the laser repetition rate, and consequently the level of thermal focusing within the laser resonator, one may control the illumination pattern and the corresponding pattern elements at a particular distance A to be either in a form of, e.g., a circle, ring or hexagon (see, e.g., also description of Fig. 9).

In exemplary embodiments, the at least one optical element may comprise at least one diffractive optical element for providing the illumination pattern, a refractive optical element for providing the illumination pattern, and/or an (optionally achromatic) metasurface for providing the illumination pattern, wherein preferably the at least one optical element may comprise a micro lens. This may yield an efficient and/or space-saving way to create the illumination pattern which may be adapted to the requirements of the specific apparatus.

In general, a diffractive optical element may comprise an optical component comprising a substrate and/or a series of (periodic) structures formed on the substrate. The (periodic) structures may, e.g., be arranged in a variety of patterns, such as gratings, lenses, prisms, cones or other shapes, depending on the desired optical function. Diffractive optical elements can be made from a variety of materials, such as glass, silicon, or plastic, using a variety of fabrication techniques, such as lithography or laser ablation. Examples of diffractive optical elements comprise diffraction gratings and/or holographic optical elements, which may be used to separate light into its component wavelengths and/or to redirect light, beam shapers and splitters, which may, e.g., be used to manipulate laser beams, and/or micro-optical components, such as lenses and prisms, which can be used in small optical systems.

The metasurface may, e.g., comprise a two-dimensional arrangement of subwavelength features, e.g., configured to manipulate the amplitude, phase, and/or polarization of electromagnetic waves. The subwavelength features may, e.g., be metallic and/or dielectric elements, and/or may be arranged in a periodic or aperiodic pattern. The metasurface may be used, for example, as a beam-steering device, a polarization converter, a wavefront modulator, a lens, and/or a hologram. The metasurface can be fabricated using various techniques, such as lithography, etching, deposition, or self-assembly, on a substrate and/or a free-standing membrane.

A micro lens may relate to a type of lens that is characterized by its small size and its ability to focus light on a very small area. Micro lenses may be beneficial, e.g., because they only require limited space and can also effectively diffract the laser light due to its dimension relative to the laser wavelength. A micro lens may be made from a variety of materials, including glass, plastic, or even semiconductor materials such as silicon. They may have a variety of shapes, such as spherical, aspherical, conical or cylindrical, depending on the specific application. They may be used to focus and/or disperse light. Micro lenses may have a high level of precision and may be manufactured by lithography or laser ablation. The at least one optical element may comprise a micro lens array comprising a plurality of identical and/or different micro lenses, wherein the plurality of micro lenses may be arranged in a regular grid pattern.

A further aspect relates to a method for shaping an illumination pattern of laser pulses for tissue treatment, the method comprising the following steps: providing at least one laser pulse and shaping an illumination pattern of the at least one laser pulse such that the illumination pattern comprises at least one pattern element with a first width of less than 300 micrometers, wherein the illumination pattern comprises a mesh comprising the at least one pattern element and wherein the mesh comprises a honeycomb lattice.

The same advantages described herein in reference to the apparatus may apply analogously to respective methods.

A further aspect of the invention relates to a method for laser treatment of a tissue surface, comprising illuminating the tissue surface with at least one laser pulse having an illumination pattern, and determining the illumination pattern by at least one optical element of an apparatus for laser treatment of the tissue surface such that the illumination pattern comprises least one pattern element with a first width of less than 300 micrometers and the effective surface coverage of the illumination pattern more than 10%.

The method may, in some examples, further comprise adjusting the illumination pattern and/or the at least one pattern element by adjusting the at least one optical element such as to shape the illumination pattern. To this end, the at least one optical element may be switchable between at least a first and a second configuration as described above. For example, the illumination pattern and/or the at least one pattern element may be varied in terms of its geometrical shape relating to, e.g., its shape (e.g., circle, ring, and/or hexagon), size, orientation, and/or intensity (distribution), e.g., by adjusting a distance between the at least one optical element and the tissue surface and/or (mutual) distances and/or (mutual) orientations between two or more components of the at least one optical element and/or by adjusting laser parameters such as, e.g., a pulse repetition rate, a laser wavelength, a laser fluence, a laser intensity, and/or a laser focus, etc.

The methods herein may be computer-implemented methods, and a computer program may be provided with instructions according to the steps of the methods. Further, any functionality described herein in reference to the system may be implemented as steps of a method and/or as instructions of a respective computer program, and vice versa.

Computer programs may be stored in any storage device, and the various method steps may be implemented in software or hardware or a combination thereof. The methods according to the present invention may be implemented in terms of a computer program which may be executed on any suitable data processing device comprising means (e.g., a memory and one or more processors operatively coupled to the memory) being configured accordingly. The computer program may be stored as computer-executable instructions on a non-transitory computer-readable medium. A data processing device may comprise one or more general purpose and/or specific purpose processors, etc.

Embodiments of the present disclosure may be realized in any of various forms. For example, in some embodiments, the present invention may be realized as a computer-implemented method, a computer-readable memory medium, or a computer system.

In some embodiments, a non-transitory computer-readable memory medium may be configured so that it stores program instructions and/or data, where the program instructions, if executed by a computer system, cause the computer system to perform a method, e.g., any of the method embodiments described herein, or, any combination of the method embodiments described herein, or, any subset of any of the method embodiments described herein, or, any combination of such subsets.

In some embodiments, a computing device may be configured to comprise a processor (or a set of processors) and a memory medium, where the memory medium stores program instructions, where the processor is configured to read and execute the program instructions from the memory medium, where the program instructions are executable to implement any of the various method embodiments described herein (or, any combination of the method embodiments described herein, or, any subset of any of the method embodiments described herein, or, any combination of such subsets). The device may be realized in any of various forms.

### 4. Short description of the figures

- Fig. 1: illustrates heat transfer mechanisms, including longitudinal and transversal cooling, in an exemplary sample, namely skin, upon a laser treatment.
- Fig. 2A: shows an exemplary delivered laser pulse sequence (an example for N = 6 is shown) and resulting temperatures at the surface of a treated sample over time.
- Fig. 2B: shows an exemplary temperature profile over depth into the treated sample after delivery of an exemplary laser pulse sequence according to Fig. 1A.
- Fig. 3: shows an exemplary illumination pattern comprising a plurality of laser spots distributed in a regular grid over a limited circular area.
- Fig. 4A: shows an exemplary delivered laser pulse cascade of four sequences (each sequence comprising N = 6 individual laser pulses) and resulting temperatures at the surface of a treated sample over time for laser treatment with a non-patterned spot-size of 7 mm.
- Fig. 4B: shows an exemplary delivered laser pulse cascade of four sequences (each sequence comprising N = 6 individual laser pulses) and resulting temperatures at the surface of a treated sample over time for laser treatment with a patterned beam profile as shown in Fig. 3 with an individual spot size of 0.85 mm.
- Fig. 5: shows an exemplary apparatus for laser treatment of a tissue surface.
- Fig. 6A: shows a schematic representation of cooling dynamics resulting from laser treatment with spot- and ring-shaped beam profiles.
- Fig. 6B: shows exemplary tessellation patterns as geometric examples for illumination patterns.
- Fig. 7: shows an exemplary optical element comprising an exemplary hexagonal micro lens array.
- Fig. 8A: shows a schematic representation of simulated illumination patterns generated by positioning the optical element at different distances from 0.50f to 1.1 f from the treated tissue surface.
- Fig. 8B: shows a schematic representation of a simulated illumination pattern generated by positioning the optical element at a distance of 2.65 f from the treated tissue surface.
- Fig. 9: shows measured illumination patterns comprising circular and/or hexagonal pattern elements as observed on a thermal paper and a thermal camera for distances between the optical element and the treated tissue surface between 0.5 f and 1.7 f.
- Fig. 10: shows a geometric comparison of a spot-pattern, a ring-pattern, and a hexagonal (honeycomb) pattern of comparable measures and a schematic representation of a transition from a spot shape to a circular shape to a hexagonal shape of a pattern element.
- Fig. 11: shows the heat profile created by a laser treatment across one cross section of the treated area for different times after delivery of the laser treatment for a patterned beam profile (top panel) and for a non-patterned beam profile (bottom panel).
- Fig. 12: shows a direct comparison of the heat profile created by a laser treatment across one cross section of the treated area right after delivery of five pulses and 100 ms after delivery of the fifth pulse for both, a patterned beam profile and for a non-patterned beam profile.

### 5. Detailed description

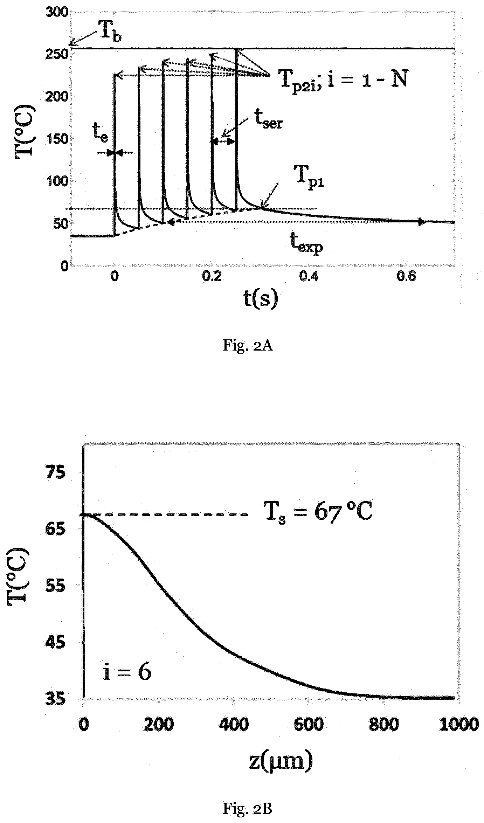

Fig. 2A shows an exemplary delivered laser pulse sequence (an example for N = 6 laser pulses is shown) and resulting temperatures at the surface of a treated sample over time.

The present invention is based on the fast superficial triggering of the epithelial surface primarily involving a short exposure process with the conventional temperature elevation of the deeper lying tissues primarily involving a long exposure process as described herein in reference to Fig. 1. This is accomplished by generating and delivering a finite series of N laser pulses to the epithelial tissue, wherein the individual laser pulses are temporally separated from each other with a serial period tₛₑᵣ which is longer than the time required for the tissue surface to appreciably cool down after the preceding laser pulse, and shorter than 5 s, preferably shorter than 2 s, and most preferably shorter than 0.5 s, in order for the deeper lying (bulk) tissue not to cool down appreciably in-between laser pulses. In such a case the tissue temperature slowly builds up during the serial delivery of laser pulses, and the long exposure process takes place. In what follows we shall denote such a serial delivery of laser pulses, which results in a combined short exposure/long exposure treatment.

Figure 2A shows a typical temporal evolution of the tissue surface temperature T during a repetitive laser pulse illumination which, as an example, uses N = 6 laser pulses with a serial period of tₛₑᵣ = 50 ms delivered by an un-patterned illumination but may analogously apply to patterned illumination.

The shown temporal evolution may be characterized by two types of peak temperatures: i) "Fast" temperature peaks Tₚ₂ᵢ, belong to individual laser pulses i (with i = 1 to N) within the sequence of N pulses: The associated intense short-duration thermal pulses resulting from individual laser pulses i, with peak temperatures (Tₘₐₓ₋ᵢ) above 70 °C at the surface lead to intense heat-shock biomodulation leading to a regeneration of deeper-lying tissues. ii) A spatial temperature distribution builds up gradually/"slowly" over the total duration of the sequence, extending several hundred microns deep into the tissue (cf. Fig. 2B), with the long-duration surface temperatures (Ts) typically below 50-70 °C. The "slow" temperature peak Tₚ₁ describes the raise of the "baseline" temperature of the tissue surface from the first to the last laser pulse, before the temperature of the tissue surface decays again in the absence of further energy delivery. Once the temperature threshold Tₚ₁ is reached, the tissue has heated up so far, that delivering further laser pulses might damage the tissue. Tₚ₁ is determined by both, transversal and longitudinal cooling as described herein. The more efficient the over-all cooling is, the later the temperature threshold Tₚ₁ is reached an more laser pulses may be delivered. The present invention provides a solution how to reach the temperature threshold Tₚ₁ later and thereby prolong the time during which laser pulses may be delivered to the tissue surface without causing damage.

The temperatures shown in Fig. 2A are measured at the centre of the illumination spot. The smooth-resurfacing treatment thus represents a unique combination of the actions of two regenerative mechanisms involving both a short-exposure and a long-exposure biochemical process.

Roughly speaking, the tissue temperature Tₚ₁ and the corresponding exposure time tₑ can be considered to be determined primarily by the long exposure process, while the tissue temperature Tₚ₂ᵢ and corresponding duration of the pulse sequence is determined primarily by the characteristics of the short exposure process. As described earlier, using a laser conforming to the illumination conditions, the amount of cell injury that is incurred during fast temperature pulsing is limited, due to the high critical temperatures under such short thermal exposure time conditions. On the other hand, the tissue injury caused by the thermal baseline "pulse" (which lasts for seconds) can be considerable already for temperatures Tₚ₁ above 55 °C to 65 °C. Therefore, controlling the slowly varying temperature elevations at the surface (and deeper within the tissue) during the laser pulse delivery is of critical importance for assuring the safety of the procedure.

Fig. 2B shows an exemplary temperature profile over depth into the treated sample after delivery of an exemplary laser pulse sequence according to Fig. 1A, illustrating that the heat delivered by the laser pulses penetrates also deeper-lying tissue.

The specific heat capacity of water which is the major constituent of tissues is Cₕ = 4.2 J/cm³ K. The cumulative fluence F_{c} = N × Fₒ that is required to heat up the tissue volume extending to a depth zₕ of the tissue for an average temperature increase of Tₕ can thus be estimated as F_{c} ≈ zₕ Cₕ Tₕ. Taking Tₕ ≈ 30 °C, we obtain F_{c} ≈ 2.5 J/cm² for zₕ = 0.2 mm, F_{c} ≈ 13 J/cm² for zₕ = 1 mm, and F_{c} ≈ 130 J/cm² for zₕ = 10 mm. Therefore, the depth zₕ of the thermally treated tissues according to the slow exposure process is determined by the cumulatively delivered fluence F_{c} = N × Fₒ over the same spot during a pulse sequence/cascade of sequences (as set on the laser system's control box). In order not to "overheat" the deeper lying tissues, it may be required to keep the cumulative fluence below F_{c} ≈ 150 J/cm², preferably below 50 J/cm², and most preferably below 15 J/cm².

Similarly, the maximal tissue surface temperature Tₚ₁ is determined by setting the serial period tₛₑᵣ and the number N of the delivered laser pulses. Measurements and calculations show that, for a full laser spot dimension of d > 1.5 mm, the time span during which the maximal temperature difference ΔTₚ₂₁ = Tₚ₂₁-T₀ of the first laser pulse drops down to ΔT_{f1} = k ΔTₚ₂₁, with k = 0.01, k = 0.02 and k = 0.03 depends on tₑ as t_{1%} ≈ 150 tₑ, t_{2%} ≈ 50 tₑ, and t_{3%} ≈ 10 tₑ, correspondingly. The goal of the serial delivery of laser pulses is that the epithelium does not get overheated while the heat is being "pumped" into the deeper lying tissues. Therefore, the serial period (tₛₑᵣ) should be longer than about 10 tₑ, preferably longer than about 50 tₑ, and most preferably longer than about 150 tₑ. On the other hand, in order that the deeper lying tissues do not cool down appreciably during the time span between two laser pulses, the serial period tₛₑᵣ should be shorter than about 3 s, preferably shorter than 1 s, most preferably shorter than 0.5 s. Further, the total duration of the laser pulse train t_{PT} = N × tₛₑᵣ should be shorter than 30 s, preferably shorter than 10 s, most preferably shorter than 5 s.

Considering that the maximal temperature increase during each laser pulse cannot be higher than a tissue boiling temperature T_{b}, this determines the lower limit for the required number of pulses as Nₘᵢₙ = F_{c}/F_{abl} with the ablation threshold fluence F_{abl}.

It should be appreciated that, for laser spot dimensions d ≤ 1.5 mm, the thermal diffusion in the lateral direction(s) during the time span tₛₑᵣ can be appreciable, which contributes to the rate of temperature decay during the time span between two laser pulses. Thus, using a small spot size allows the delivery of higher fluences at shorter serial times tₛₑᵣ. When irradiation is performed in a "full spot" manner a typical laser spot size S' has a diameter d larger than 5 mm, since having to cover the larger over-all treatment area with a small spot size would be impractical. The thermal relaxation time (TRT) due to the diffusion of heat from a uniformly heated circular spot of diameter d is TRT_{circle} = d²/16 D, wherein D is the skin's heat diffusivity (D ~ 0.1 mm²/s). Since for d ≥ 5 mm, the TRT_{circle} is longer than 15 s, the transverse heat diffusion does not have a key role in full-spot smooth resurfacing treatments. From this, it may be derived that patterning the illumination, e.g., in form of multiple smaller circular laser spots may be beneficial.

Accordingly, the energy may preferably be delivered to the tissue in a "patterned" shape, wherein the laser beam irradiates a number of individual spots S within the treatment area S' Each spot S having the size (e.g., diameter) d is separated from a neighbouring spot by the distance L. Fig. 3 shows such exemplary patterned illumination pattern 300 comprising a plurality of M laser spots S distributed in a regular grid over a limited circular area S'. The spot size d and the distance x are chosen such that the spot size d is in the range of 0.8-1 mm, with L = 2 mm, so that the tissue coverage T_{C} = (M × area(S))/area(S') (in %) may be in the range of 25% ≤ T_{C} ≤ 40 %. These parameters ensure that, during the time span between two laser pulses (i.e., during tₛₑᵣ), the thermal diffusion in the lateral direction spreads the heat which is generated by the laser radiation away from a localized spot S towards the surroundings of the spot, thus effectively spatially homogenizing the slowly varying temperature Tₚ₁ across the area S'. The TRT of the pattern comprising multiple laser spots (see Fig. 3) is in the range of 0.4-0.6 s which is comparable or shorter than a typical duration of the smooth-resurfacing sequence. Thus, intense heat shocks with short exposure time can be delivered to the localized spots S, without causing long-term overheating of the treatment area S'. This way, it is possible to create an intense heat "needling" on about 30% of the surface of the tissue, whereas the thermal injury in deeper layers is approximately homogeneous (in the lateral direction), since, in the deeper layers, the thermal diffusion spreads the heat in all directions.

There are several possibilities how a plurality of spots within the treatment area may be generated. First, one can use a screen with various holes in front of the laser handpiece. However, if the fluence of laser radiation becomes too large, the screen which blocks the laser radiation can become too hot. Alternatively, an array of lenses can be used for creating a pattern of distinct spots within the treatment area, wherein the laser beam illuminates the array of lenses. It should be noted that the array of lenses can comprise one layer of lenses which are transversally arranged with respect to the optical axis or, the array of lenses can comprise more than one layer of lenses which follow each other on the optical axis. Finally, diffraction optics can be used, in order to generate the pattern of distinct spots within the treatment area by using interference effects.

To demonstrate the influence of radial heat diffusion for small diameter beam sizes we carried out a limited numerical analysis using a 3D cylindrical coordinate system model. In detail, a comparison of the tissue response of the two aforementioned apparatuses, namely a first apparatus with un-patterned illumination (cf. Fig. 4A) and a second apparatus with an illumination pattern as shown in Fig. 3 (cf. Fig. 4B) are shown to illustrate the advantageous effect of patterning the illumination of the tissue surface to be treated by the apparatus. The temporal profiles show the skin surface temperature resulting during a sequence with N = 24 laser pulses:
Fig. 4A shows an exemplary delivered laser pulse cascade 420a of four sequences 410a (each sequence comprising N = 6 individual laser pulses) and resulting temperatures at the surface of a treated sample over time for laser treatment with a non-patterned spot-size of 7 mm. It shows calculated temporal profile of the skin surface temperature during a sequence of four smooth mode pulse sequences each consisting of 6 laser pulses (resulting in N = 4 × 6 = 24 laser pulses) for Fₛ = 4.9 J/cm².

Fig. 4B shows an exemplary delivered laser pulse cascade 420b of four sequences 410b (each sequence comprising N = 6 individual laser pulses) and resulting temperatures at the surface of a treated sample over time for laser treatment with a patterned beam profile as shown in Fig. 3 with an individual spot size of 0.85 mm. It shows calculated temporal profile of the skin surface temperature during a sequence of four smooth mode pulse sequences each consisting of 6 laser pulses (resulting in N = 4 × 6 = 24 laser pulses) for Fs = 14.7 J/cm².

As can be seen from Figs. 4A and 4B, for the same final sequence temperature (Tₛ = 60 °C) (and resulting deep tissue response), the high temperature peaks (T_{max-N}) are approximately 30% higher for the patterned illumination (cf. Fig. 4B). A damage integral calculation shows that this difference results in 2 times stronger (in terms of the damage integral) patterned thermal "needling" of the tissue. Altogether, patterning allows to substantially increase the high-temperature stimulation of the treated tissue surface while keeping the heating of lower-lying layers relatively low. Thereby, tissue damage may be avoided while simultaneously triggering substantial regenerative responses of the treated tissue as said response depends on the peak temperatures T_{max-N}.

This illustrates that generally, as a first step, patterned instead of full-spot illumination may improve the efficiency of according apparatuses for tissue treatment. However, the question arises whether a better pattern structure than that used in the patterned illumination (see Fig. 3) can be found where a similar coverage T_{C} would be achieved with even faster transverse cooling, and therefore with even more intense heat shock triggering. Indeed, circular spots, nevertheless, do not stipulate the ideal illumination patterns to optimize such apparatuses, as explained in the following:
Fig. 5 shows an exemplary apparatus for laser treatment of a tissue surface. The invention may comprise an improved patterned illumination in comparison to the patterned illumination presented in Fig. 3, with a goal to achieve faster transverse cooling, and therefore more intense heat shock triggering, and as well a more energy efficient patterned illumination of the tissue surface. An exemplary apparatus for laser treatment of a tissue surface (Fig. 5) may comprise at least one optical element for illuminating the tissue surface with at least one laser pulse having an illumination pattern; wherein the at least one optical element is configured such that the illumination pattern comprises at least one pattern element with a first width of less than 300 micrometers. The exemplary apparatus of Fig. 5 comprises a laser, delivery optics, and an optical element. The laser light emitted by the laser is in the example of Fig. 5 guided to the optical element by the delivery optics, e.g., an optical fiber, mirrors, and/or gratings, etc. The optical element may then be positioned at a distance A relative to the tissue surface to be treated and create an illumination pattern thereon, e.g., as described herein.

Fig. 6A shows a schematic representation of cooling dynamics resulting from laser treatment with spot- and ring-shaped beam profiles. A circular beam spot, e.g., with a Gaussian or top hat intensity profile, results in an essentially circular heated area 510 on the treated tissue surface. Transversal heat transfer may predominantly occur from the edges of the circular heated area 510 in an outward radial direction to the surrounding, as illustrated by the dashed arrows. In contrast, a ring-shaped illumination results in a ring-shaped heated area 520 on the treated tissue surface. Transversal heat transfer may therein predominantly occur from the edges of the ring-shaped heated area 520 in an outward radial direction and an inward radial direction to the surrounding, as illustrated by the dashed arrows.

This illustrates schematically, that the transversal heat transfer may substantially be enhanced when illumination patterns other than spot-like patterns are utilized. This is further substantiated by simply calculating the TRT for the two illustrative examples above: Due to the diffusion of heat from a uniformly heated circular spot of diameter d can be calculated to be: TRT_{circle} = d²/16 D. Since for d ≥ 5 mm, the TRT is longer than 15 s, the transverse heat diffusion does not have an important role in full-spot smooth resurfacing treatments. Consider a pattern consisting of rings having the same area as the circles of the circular pattern as, e.g., shown in Fig. 3. Taking d of the circle to be equal to 1 mm then TRT_{circle} = d²/ 16D = 0,63 s. An exemplary ring with the same area has the radius equal to r = 0.9 mm and dₛ of the ring equal to dₛ = 0,16 mm. Taking into account that the width dₛ of the ring is significantly smaller than its radius r then the TRT_{ring} of the ring can be approximately calculated to be TRT_{ring} = dₛ²/D = 0.26 s. A comparison of the TRTs of the two shapes of the same area shows that the TRT of the ring is approximately 2.4-times shorter in comparison with the TRT of the circle.

While a circular beam shape may therefore be preferable from a strictly mathematical point of view, a pattern comprising only circular elements cannot cover an area regularly without creating unwanted irregular gaps. Therefore, it may be altogether preferable to utilize illumination patterns comprising linear pattern elements along the boundary lines of tessellation patterns, i.e., where the laser radiation is present predominantly on the perimeters of the covering shapes of the patterns. Such pattern may combine two preferred aspects described herein: a reduced surface coverage in general and an optimization of the area-to-perimeter ratio as described for the exemplary comparison of circular and ring-shaped pattern elements.

Fig. 6B shows exemplary tessellation patterns 610, 620, 630 as geometric examples for illumination patterns.

The illumination patterns illustrated schematically in Fig. 6B comprise lines. For each line it may be understood that the illumination intensity may, e.g., exhibit a Gaussian intensity distribution at the illuminated surface in a direction perpendicular to the course of the line at each point. Analogously, points may represent an illumination intensity at the substrate with a 2D-Gaussian intensity profile, like e.g., typical laser spots.

The first example of Fig. 6 shows a triangular tessellation 610. The triangular tessellation pattern 610 is a repeating pattern of equilateral triangles that fill a two-dimensional plane without any gaps or overlaps. The tessellation is characterized by its uniformity, where each triangle in the pattern has three congruent sides and three equal inner angles of 60°, and by the way in which the triangles are arranged in a repeating pattern, i.e., at each corner point, six triangles meet. The mesh based on the triangular tessellation pattern 610 comprises straight lines 613, 614, 615 connecting the corner points of the triangles. Said lines may, in other examples, be curved in any regular or irregular way.

The triangular pattern 610 may, e.g., be defined by the two lattice vectors 611, 612. The three straight lines 613, 614, 615 form the basis. Together, the lattice vectors 611, 612 and the basis 613, 614, 615 mathematically stipulate the whole triangular pattern.

The exemplary inset, which may apply to the shown triangular pattern or not and/or to other patterns or not, shows that the lines of the pattern 610, corresponding to a spatial intensity distribution on the treated tissue surface, are no sharp but blurred lines, e.g., with a Gaussian intensity profile defining the first width of the pattern elements, i.e., a line in this example.

The second example of Fig. 6 shows a square tessellation 620. The square tessellation pattern 620 is a repeating pattern of equilateral squares that fill a two-dimensional plane without any gaps or overlaps. The tessellation is characterized by its uniformity, where each square in the pattern has four congruent sides and four equal inner angles of 90°, and by the way in which the squares are arranged in a repeating pattern, i.e., at each corner point, four triangles meet. The mesh based on the square tessellation pattern 620 comprises straight lines 623, 624 connecting the corner points of the squares. Said lines may, in other examples, be curved in any regular or irregular way.

The square pattern 620 may, e.g., be defined by the two lattice vectors 621, 622. The two straight lines 623, 624 form the basis. Together, the lattice vectors 621, 622 and the basis 623, 624 mathematically stipulate the whole square pattern.

The exemplary inset, which may apply to the shown triangular pattern or not and/or to other patterns or not, shows that the lines of the pattern 620, corresponding to a spatial intensity distribution on the treated tissue surface, may comprise a series of spots, e.g., each with a 2D-Gaussian intensity profile defining the first width of the pattern elements, arranged along the extension of the line.

The third example of Fig. 6 shows a hexagonal tessellation 630. The hexagonal tessellation pattern 630 is a repeating pattern of equilateral hexagons that fill a two-dimensional plane without any gaps or overlaps. The tessellation is characterized by its uniformity, where each hexagon in the pattern has six congruent sides and six equal inner angles of 120°, and by the way in which the hexagons are arranged in a repeating pattern, i.e., at each corner point, three hexagons meet. The mesh based on the hexagonal tessellation pattern 630 comprises straight lines 633, 634, 635 connecting the corner points of the squares. Said lines may, in other examples, be curved in any regular or irregular way.

The hexagonal pattern 620 may, e.g., be defined by the two lattice vectors 631, 632. The three straight lines 633, 634, 635 form the basis. Together, the lattice vectors 631, 632 and the basis 633, 634, 635 mathematically stipulate the whole hexagonal pattern.

The examples 610, 620, 630 of Fig. 6B relate to infinite 2D patterns. The illumination pattern of the present invention may relate to finite portions of such exemplary or other patterns, e.g., a circular portion and/or a portion of any other shape.

The regular tessellation pattern that may be seen as the closest to a circular pattern may be the hexagonal tessellation pattern 630 as it is the most efficient way to maximize area while minimizing perimeter. As can be easily shown, when a triangle and hexagon have the same perimeter, the hexagon has much larger area.

Fig. 7 shows an exemplary optical element comprising an exemplary hexagonal micro lens array, as used in some of the experiments and simulations described herein. The hexagonal micro lenses of the example of Fig. 7 are arranged such that the hexagonal micro lenses comprise only next neighbors with parallel faces and the distance between the faces to all of the next neighbors are identical. The exemplary patterned illumination described herein may be, e.g., achieved using such optical element consisting of a hexagonal micro lens array, preferably a staggered hexagonal micro lens array with approximately tessellation coverage. Each of the micro lenses has a certain dimension, defined as the lens pitch (Lₚ) and a certain radius resulting in an optical focus of the lens (f)

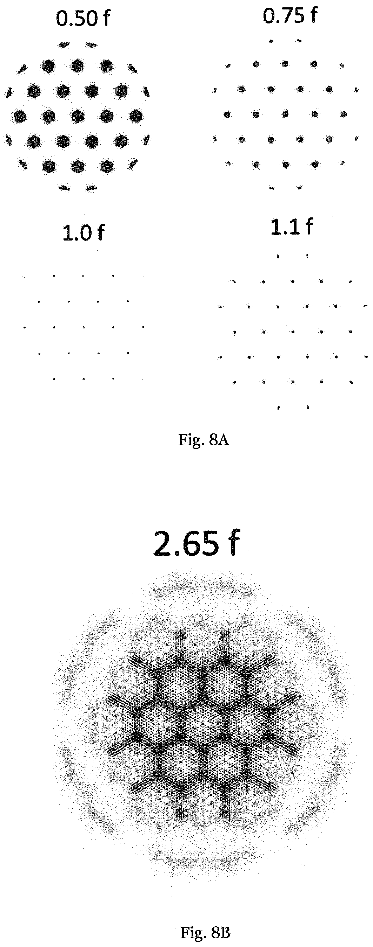

Fig. 8A shows a schematic representation of simulated illumination patterns generated by positioning the optical element at different distances A from 0.50f to 1.1 f from the treated tissue surface. The underlying computer simulations of the wavefront propagation consider an exemplary hexagonal micro lens array with a lens pitch Lₚ = 1.6 mm and a focal length f = 131 mm (radius 60 mm), and a laser source with a flat wavefront and an ideal top hat spatial profile at the location of the micro lens array, and wavelength of 630 nm. With increasing distance A the pattern elements transform from hexagonal "rings" (see illumination pattern for A = 0.5 f in Fig. 8A) into circular rings with decreasing diameter until A = f (see illumination patterns for A = 0.75 f and A = 1.0 f in Fig. 8 A). The distances between the pattern elements remains essentially unaffected by A in the example of Fig. 8A (and also in the example of Fig. 8B).

For distances A longer then f the diameter of the circular rings of the illumination pattern start to increase (See the pattern for A = 1.1 f in Fig. 8A) until they "merge" together into hexagonal pattern at a distance of about A = 2.65 f. Fig. 8B shows a schematic representation of a simulated illumination pattern generated by positioning the optical element at a distance of 2.65 f from the treated tissue surface. In the exemplary configuration of Fig. 8B, the illumination pattern comprises an intensity distribution that essentially corresponds to a hexagonal honeycomb pattern. The pattern elements may be seen as face-sharing hexagons with a first width corresponding to the contour width of the hexagons. The illumination pattern is spatially limited an essentially circular area and comprises essentially 19 face-sharing hexagons, wherein a central hexagon comprises six neighboring hexagons at all of its faces and the six neighboring hexagons of the central hexagon share each face with one hexagonal pattern element. This yields an essentially hexagonal shape of the area wherein the hexagonal pattern elements are present, wherein at the margins, the illumination pattern smears out. The simulations also show that when the wavefront of the illumination at the location of the optical element is not flat but has a certain divergence then the distance at which the hexagonal pattern develops gets shorter towards larger divergence.

Fig. 9 shows measured illumination patterns comprising circular and/or hexagonal pattern elements as observed on a thermal paper and a thermal camera for distances between the optical element and the treated tissue surface between 0.5 f and 1.7 f. It shows an exemplary embodiment, wherein the laser wavelength was 2940 nm (Er:YAG laser), and the optical element consisting of a micro lens array with a lens pitch Lₚ = 1.6 mm and a focal length f = 131 mm (radius 60 mm).

The top row of Fig. 9 shows measured illumination patterns comprising circular and/or hexagonal pattern elements as observed on a thermal paper for distances between the optical element and the treated tissue surface of 0.5 f, 0.9 f, 1.15 f, and 1.7 f. The bottom row shows measured illumination patterns comprising circular and/or hexagonal pattern elements as observed by a thermal camera for distances between the optical element and the treated tissue surface of 0.9 f, 1.15 f, and 1.7 f. Essentially, the top and bottom row of Fig. 9 are mutually consistent and illustrate the following:
As can be seen from Fig. 9, the hexagonal (honeycomb) pattern developed at a shorter distance as calculated for an exemplary flat wavefront with ideal top hat profile, due to the more realistic multi-mode Er:YAG laser beam emitted, e.g., by the utilized Fotona Dynamis laser.

The parameters of the laser beam at the exit mirror of the Dynamis Er:YAG laser were as follows: beam diameter: 3,2 mm, wavefront radius of curvature: flat. With increasing thermal lensing in the laser rod with increasing pulse repetition rates from 2-40 Hz the beam quality ratio M² increased from M²= 16 to M²= 24, and the half angle divergence of the laser bean increased from 9.4 mrad (at M² = 16) to 15 mrad (at M² = 25).

The laser beam parameters at the location of the micro-lens array optical element were as follows: beam diameter of 6,4 mm (substantially independent of M²), and half angle divergence from 7 mrad (at M² = 16) to 8,5 mrad (at M² = 25). The diverging wavefront radius varied from 615 mm (at M² = 16) to 730 mm (at M² = 25).

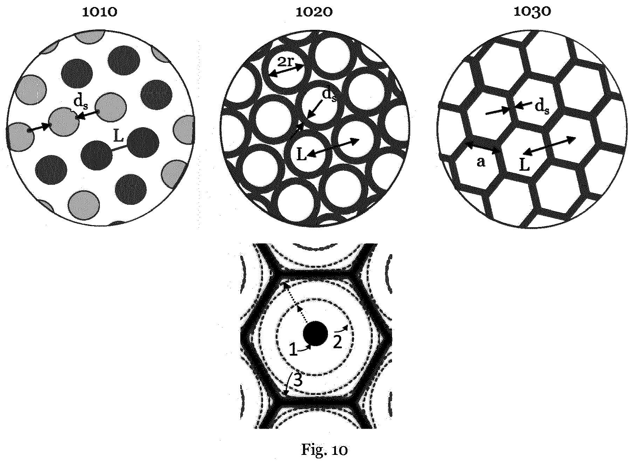

It is to be noted that the honeycomb-patterned laser intensity represents only a limiting example of a general staggered distribution of optical patterns. Therefore, this exemplary embodiment may represent a stable optical design solution where by adjusting the distance and/or laser divergence, the apparatus can be made to operate either in an illumination pattern of circular spots in a staggered organization (cf. illumination pattern 1010 of Fig. 10), or circular rings (cf. illumination pattern 1020 of Fig. 10) or hexagons (cf. illumination pattern 1030 of Fig. 10). Depending on the desired effect on the tissue surface, each of the three patterned illuminations has certain advantages. The shape with the largest area for a given perimeter is a circle. The ring shape may result in an improved thermal cooling, and the hexagonal shape may cover the complete surface in an energetically most efficient manner. The distance can be changed, for example, by using a different spacer. Since the laser beam output divergence increases with the thermal lensing within the Er:YAG laser rod, the laser's divergence may, e.g., be adjusted by adjusting the laser input power by increasing the laser pulse repetition rate. In the experiments the tested laser repetition rates used were in the range of 10-40 Hz. The patterns shown in Fig. 9 may, e.g., be achieved for a pulse repetition rate of 40 Hz.

Fig. 10 shows a geometric comparison of a spot-pattern 1010, a ring-pattern 1020, and a hexagonal (honeycomb) pattern 1030 of comparable measures, e.g., created as explained in reference to Fig. 9. In all three examples, L indicates the length of the lattice vectors, dₛ indicates the width of the respective pattern element (a spot or a line, respectively). In the ring-pattern 1020, 2r indicates the diameter of one ring and in the honeycomb pattern 1030, a indicates the distance between two neighboring corner points of a hexagon. The bottom panel of Fig. 10 shows a schematic comparison of how adjusting the laser parameters as explained herein may result in changes from an illumination pattern comprising 1) circular spots in a staggered organization (cf. illumination pattern 1010 of Fig. 10) to 2) circular rings (cf. illumination pattern 1020 of Fig. 10) and/or 3) hexagons (cf. illumination pattern 1030 of Fig. 10).In the example of Fig. 10, the micro lenses are arranged in a staggered triangular grid-like pattern. The micro lens array may be configured to generate ring-shaped pattern elements on the tissue surface. For example, as shown in Fig. 10, each ring-shaped pattern element has six nearest neighbors being ring-shaped pattern elements as well. Thereby, theoretically, such micro lens array may generate a ring-pattern. However, it was observed that the ring-shaped pattern elements 810 merge and form a hexagonal honeycomb pattern. Considering optical designs which would result in a honeycomb-patterned laser intensity it is worth noting that the honeycomb pattern represents only a limiting situation. Actually, the shape with the largest area for a given perimeter is a circle. Therefore, a stable optical design solution generates circular rings which under limiting laser output parameters do not dissolve but instead naturally transform into rounded hexagons. The dashed arrows in Fig. 10 illustrate the transition from the spot shape to the ring-shape and from the ring-shape to the hexagonal pattern, respectively. This illustrates how starting with a circular shape in a precise staggered organization allows the transformation into the rounded hexagonal shape near the triple junction between the neighboring circular cells. This further allows to cover the complete surface in an energetically efficient manner.

Exemplary patterns may, e.g., comprise spot patterns with spot diameters dₛ in the range from 0.01 to 0.3 mm, preferably from 0.1 to 0.2 mm. Exemplary honeycomb patterns may comprise hexagons with edge lengths a in the range from 0.1 mm to 3 mm, preferably from 0.05 mm to 2 mm, more preferably from 0.75 mm to 1.25 mm and/or line widths in the range from 0.065 mm to 0.3 mm, preferably from 0.1 mm to 0.2 mm yielding surface coverages of 10% to 52% and 16% to 33%, respectively. Exemplary examples are summarized in table 1. An analysis of the three geometrical patterns depicted in Fig. 10 reveals the following corresponding coverages and TRTs (see Table 1). Table 1 essentially illustrates that the HC honeycomb pattern allows an advantageous coverage of the treated tissue surfaces yielding a reduced transverse TRT compared to spot patterns (cf. "Spot 1" of table 1), hexagonal spot patterns (cf. "HC spot" of table 1), patterns comprising circular pattern elements (cf. "HC circles" of table 1), and full spot illumination (cf. "full spot" of table 1). As mentioned, e.g., in reference to Fig. 8B, the exemplary embodiments of table 1 relate may, e.g., comprise micro lens arrays configured to generate illumination patterns with 19 pattern elements, e.g., arranged as shown in Fig. 8A, 8B, etc.

**Table 1**

| | | **HC circles** | **HC rings** | **HC honeycomb** | **Full spot** |
|---|---|---|---|---|---|
| **dₛ (mm)** | | 1 | 0,13 | 0,16 | 7 |
| **N spots** | | 19 | 19 | 19 | 1 |
| **r or a (mm)** | | | 0,87 | 1,15 | |
| **S (mm²)** | | 0,79 | 0,76 | 1,05 | 38,465 |
| **Stot (mm²)** | | 14,92 | 14,50 | 9,99 | 38,465 |
| **Ratio** | | 2,58 | 2,65 | 3,85 | 1 |
| **Coverage (%)** | | 39% | 38% | 26% | 100% |
| **Transverse TRT (s)** | | 0,57 | 0,61 | 0,23 | 27,84 |

Comparing pattern "HC circles" and "HC rings" (cf. e.g., patterns 1010 and 1020 of Fig. 10), it is evident that for the circular pattern the TRT is essentially as fast as for a spot pattern with a similar surface coverage of 39% or 38%. This is because for the closely packed rings 1020 if Fig. 10, the effective width of the ring may be taken to be equal to 2dₛ since the neighboring rings are touching or close to each other-effectively slowing down heat diffusion.

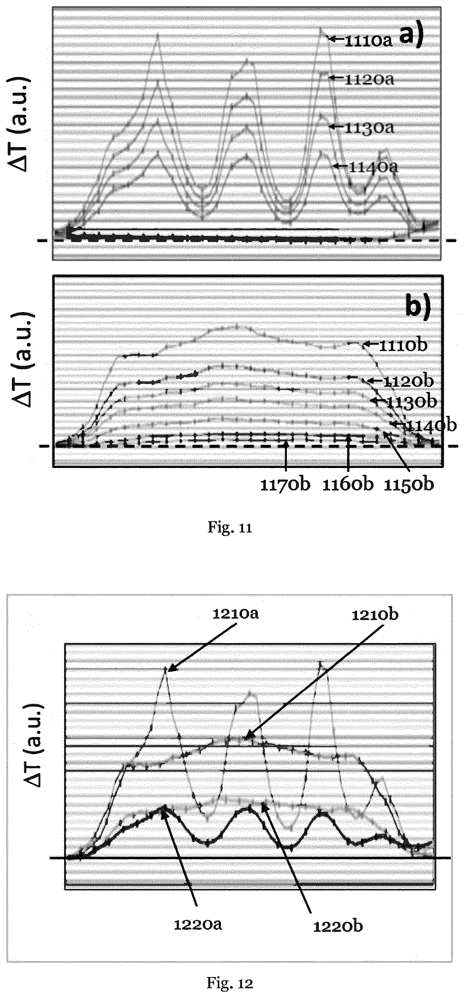

However, the "HC honeycomb" pattern with well-resolved hexagons (cf. e.g., pattern 1030 of Fig. 10) clearly has a reduced surface coverage of 26% vs. ≥ 38% and outcompetes the other patterns of table 1 in terms of TRT (0.23 s vs. ≥ 0.57 s). The analysis shows that HC-patterned apparatuses may be better suited for the maximal heat shock triggering effect procedures compared to full spot or spot-patterned apparatuses (cf. "full spot" table 1 for reference). In summary, this indicates that both, reducing the surface coverage and optimizing the pattern in terms of its area-to-perimeter ration help to improve the TRT, which in turn may improve the thermal response triggered in the treated tissue surface as shown in the following:
The top panel of Fig. 11 shows the heat profile created by a laser treatment across the treated area for different selected times after delivery of the laser treatment for a hexagonally/honeycomb-patterned illumination pattern (cf. table 1). Essentially, the temporal evolution of the temperature profile for the hexagonally patterned illumination from the moment of illumination 1110a reduces in amplitude over time as illustrated by further later snapshots of the temperature profile 1120a, 1130a, 1140a.

The bottom panel of Fig. 11 shows the heat profile created by a laser treatment across the treated area for different selected times after delivery of the laser treatment for a un-patterned, full spot illumination pattern (cf. Table 1). Essentially, the temporal evolution of the temperature profile for the un-patterned, full spot illumination from the moment of illumination 1110b reduces in amplitude over time as illustrated by further later snapshots of the temperature profile 1120b, 1130b, 1140b, 1150b, 1160b, 1170b.

Both panels show an exemplary qualitative evolution of the heat distribution in the treated tissue over time after illumination. The main difference between the two illumination is that the temperature profile resulting from hexagonally/honeycomb-patterned illumination 1110a, 1120a, 1130a, 1140a yields peaks as a function of position at all times while the full spot illumination 1110b, 1120b, 1130b, 1140b, 1150b, 1160b, 1170b yields a heat plateau as a function of position at all times. Based on this basic understanding of the dynamics, Fig. 12 shows an illustrative comparison of the cooling dynamics in these two scenarios:
In detail, Fig. 12 shows a direct comparison of four heat profiles 1210a, 1210b, 1220a, 1220b in terms of a change in temperature ΔT induced by the illumination across one intersecting line through the illuminated area on the tissue surface. The shown heat profiles 1210a, 1210b, 1220a, 1220b are created by a laser treatment for both, a patterned illumination (a honeycomb pattern, cf. e.g., "HC honeycomb" of table 1) and for a non-patterned illumination for reference:
The heat profile created by illumination with a honeycomb pattern right after illumination by five honeycomb-patterned laser pulses 1210a drops within the heat profile 1220a. Both heat profiles 1210a, 1220a created by the same honeycomb illumination exhibit peaks at the positions of the pattern elements and the predominant change from the initial heat profile 1210a to the heat profile after 100 ms 1220a is the amplitude drop. Two further heat profiles created by non-patterned illumination, i.e., full spot illumination, right after illumination by five full spot illumination laser pulses 1210b and 100 ms after illumination 1220b (with a diminished amplitude compared to heat profile 1210b) are shown for comparison.

The displayed heat profiles 1210a, 1210b, 1220a, 1220b are normalized such that the 100 ms heat profiles for honeycomb patterned illumination 1220a and for full spot illumination 1220b essentially exhibit the same amplitude. This long-term amplitude may be seen as a measure for over-all heating of the illuminated tissue. Thereby, the comparison of Fig. 12 shows that significantly higher peak temperatures may be reached by honeycomb patterned illumination than with full spot illumination for essentially the same over-all, long-term heating of the illuminated tissue. The honeycomb pattern heat profile right after illumination 1210a comprises peaks that significantly exceed the maximum temperature of the heat profile right after illumination created by full spot illumination 1210b. This may be associated with the following differences in the heat transfer dynamics:
Within 100 ms, the full spot illumination amplitude drops by about 42% while the honeycomb illumination amplitude drops faster, by 72%. Since for full spot illumination the contribution of the transverse diffusion is negligible, the observed cooling rate for full spot illumination can be attributed mainly to the longitudinal heat diffusion. The difference in the cooling rates of 30% is therefore a result of the enhanced transverse heat diffusion of the HC pattern. This additional rate of cooling by 30% in 100 ms translates into a transverse TRT of approximately 0.3 s, in good agreement with the estimated TRT for HC long of 0.23 s (see Table 1).

## Claims

1. An apparatus (100) for laser treatment of a tissue surface (110, 120), the apparatus (100) comprising:
at least one optical element for illuminating the tissue surface (110, 120) with at least one laser pulse (410a, 410b) having an illumination pattern (610, 620, 630);
wherein the at least one optical element is configured such that:
the illumination pattern (630) comprises at least one pattern element (633, 634, 635) with a first width of less than 300 micrometers; and
an effective surface coverage of the illumination pattern (630) is more than 10%;
a) wherein the illumination pattern (630) comprises a lattice comprising the at least one pattern element, and wherein the lattice comprises a lattice of closely packed rings, the neighboring rings touching each other, or a lattice of closely packed rounded hexagonal rings, the neighboring rounded hexagonal rings touching each other, or
b) wherein the illumination pattern (630) comprises a mesh comprising the at least one pattern element (633, 634, 635); and
wherein the mesh comprises a honeycomb lattice.

2. The apparatus (100) of claim 1, wherein a pulse energy of the at least one laser pulse (410a, 410b) is within a range such that the corresponding fluence on the tissue surface (110, 120) corresponds to a non-ablative treatment of the tissue surface (110, 120).

3. The apparatus (100) of claim 1 or 2, wherein the at least one pulse has a pulse length shorter than 2 ms, preferably shorter than 900 microseconds.

4. The apparatus (100) of any of claims 1 - 3, wherein the apparatus (100) further comprises a laser source, preferably an infrared laser source, more preferably an Er:YAG laser source.

5. The apparatus (100) of any of claims 1 - 4, wherein the tissue is predetermined and the apparatus (100) is configured to deliver the at least one laser pulse (410a, 410b), such that each of the at least one laser pulse (410a, 410b) heats the tissue surface (110, 120) to a temperature above 70°C.

6. The apparatus (100) of any of claims 1 - 5, further comprising:
means for receiving a user input regarding the tissue surface (110, 120) to be treated; and
means for automatically adapting at least one parameter of the at least one laser pulse (410a, 410b) based at least partly on the user input.

7. The apparatus (100) of any of claims 1 - 6, wherein the at least one pattern element (633, 634, 635) has a line shape and/or forms at least a part of a line shape.

8. The apparatus (100) of any of claims 1 - 7, wherein the first width corresponds to a width of the line shape and wherein the line shape has a length (L) of at least 4 times, preferably at least 10 times of the first width.

9. The apparatus (100) of any of claims 1 - 8, wherein the lattice comprises at least one lattice vector with a length (L) in a range from 0.1 mm to 10 mm, 0.5 mm to 5 mm, or 1 mm to 3 mm.

10. The apparatus (100) of any of claims 1 - 9, wherein the effective surface coverage of the illumination pattern (630) is less than 50%, less than 40%, or less than 30%.

11. The apparatus (100) of any of claims 1 - 10, wherein the illumination pattern (630) forms boundary lines of a tessellation pattern, preferably a regular tessellation pattern.

12. The apparatus (100) of any of claims 1 - 11, wherein the at least one optical element is switchable between at least a first and a second configuration;
wherein the at least one pattern element comprises a first geometrical shape when the at least one optical element is in the first configuration and wherein the at least one pattern element comprises a second geometrical shape when the at least one optical element is in the second configuration, wherein the first geometrical shape is different from the second geometrical shape.

13. The apparatus (100) of any of claims 1 - 12, wherein the at least one optical element comprises at least one diffractive optical element, at least one refractive optical element, and/or at least one metasurface for providing the illumination pattern (630), wherein preferably the at least one optical element comprises a micro lens.

14. A non-therapeutic method for shaping an illumination pattern (630) of laser pulses (410a, 410b) for tissue treatment, the method comprising the following steps:
providing at least one laser pulse (410a, 410b);
shaping an illumination pattern (630) of the at least one laser pulse (410a, 410b) such that the illumination pattern (630) comprises at least one pattern element (633, 634, 635) with a first width of less than 300 micrometers; and
wherein an effective surface coverage of the illumination pattern (630) is more than 10%;
a) wherein the illumination pattern (630) comprises a lattice comprising the at least one pattern element, and wherein the lattice comprises a lattice of closely packed rings, the neighboring rings touching each other, or a lattice of closely packed rounded hexagonal rings, the neighboring rounded hexagonal rings touching each other, or
b) wherein the illumination pattern (630) comprises a mesh comprising the at least one pattern element (633, 634, 635); and
wherein the mesh comprises a honeycomb lattice.

## Patentansprüche

1. Vorrichtung (100) zur Laserbehandlung einer Gewebeoberfläche (110, 120), wobei die Vorrichtung (100) umfasst:
mindestens ein optisches Element zum Beleuchten der Gewebeoberfläche (110, 120) mit mindestens einem Laserpuls (410a, 410b) mit einem Beleuchtungsmuster (610, 620, 630);
wobei das mindestens eine optische Element so konfiguriert ist, dass:
das Beleuchtungsmuster (630) mindestens ein Musterelement (633, 634, 635) mit einer ersten Breite von weniger als 300 Mikrometern umfasst; und
eine effektive Oberflächenabdeckung des Beleuchtungsmusters (630) mehr als 10 % beträgt;
a) wobei das Beleuchtungsmuster (630) ein Gitter umfasst, das das mindestens eine Musterelement umfasst, und wobei das Gitter ein Gitter aus dicht gepackten Ringen, wobei die benachbarten Ringe einander berühren, oder ein Gitter aus dicht gepackten abgerundeten sechseckigen Ringen, wobei die benachbarten abgerundeten sechseckigen Ringe einander berühren, umfasst, oder
b) wobei das Beleuchtungsmuster (630) ein Netz umfasst, das das mindestens eine Musterelement (633, 634, 635) umfasst; und
wobei das Netz ein Wabengitter umfasst.

2. Vorrichtung (100) nach Anspruch 1, wobei eine Pulsenergie des mindestens einen Laserpulses (410a, 410b) innerhalb eines Bereichs liegt, so dass die entsprechende Fluenz auf der Gewebeoberfläche (110, 120) einer nicht ablativen Behandlung der Gewebeoberfläche (110, 120) entspricht.

3. Vorrichtung (100) nach Anspruch 1 oder 2, wobei der mindestens eine Puls eine Pulslänge von weniger als 2 ms, vorzugsweise weniger als 900 Mikrosekunden hat.

4. Vorrichtung (100) nach einem der Ansprüche 1 bis 3, wobei die Vorrichtung (100) ferner eine Laserquelle, vorzugsweise eine Infrarotlaserquelle, noch bevorzugter eine Er:YAG-Laserquelle umfasst.

5. Vorrichtung (100) nach einem der Ansprüche 1 bis 4, wobei das Gewebe vorbestimmt ist und die Vorrichtung (100) konfiguriert ist, um den mindestens einen Laserpuls (410a, 410b) abzugeben, so dass jeder des mindestens einen Laserpulses (410a, 410b) die Gewebeoberfläche (110, 120) auf eine Temperatur über 70 °C erwärmt.

6. Vorrichtung (100) nach einem der Ansprüche 1 bis 5, ferner umfassend:
Mittel zum Empfangen einer Benutzereingabe bezüglich der zu behandelnden Gewebeoberfläche (110, 120); und
Mittel zum automatischen Anpassen mindestens eines Parameters des mindestens einen Laserpulses (410a, 410b) basierend mindestens teilweise auf der Benutzereingabe.

7. Vorrichtung (100) nach einem der Ansprüche 1 bis 6, wobei das mindestens eine Musterelement (633, 634, 635) eine Linienform aufweist und/oder mindestens einen Teil einer Linienform bildet.

8. Vorrichtung (100) nach einem der Ansprüche 1 bis 7, wobei die erste Breite einer Breite der Linienform entspricht und wobei die Linienform eine Länge (L) von mindestens dem 4-fachen, vorzugsweise mindestens dem 10-fachen der ersten Breite hat.

9. Vorrichtung (100) nach einem der Ansprüche 1 bis 8, wobei das Gitter mindestens einen Gittervektor mit einer Länge (L) in einem Bereich von 0,1 mm bis 10 mm, 0,5 mm bis 5 mm oder 1 mm bis 3 mm umfasst.

10. Vorrichtung (100) nach einem der Ansprüche 1 bis 9, wobei die effektive Oberflächenabdeckung des Beleuchtungsmusters (630) weniger als 50 %, weniger als 40 % oder weniger als 30 % beträgt.

11. Vorrichtung (100) nach einem der Ansprüche 1 bis 10, wobei das Beleuchtungsmuster (630) Grenzlinien eines Mosaikmusters, vorzugsweise eines regelmäßigen Mosaikmusters, bildet.

12. Vorrichtung (100) nach einem der Ansprüche 1 bis 11, wobei das mindestens eine optische Element zwischen mindestens einer ersten und einer zweiten Konfiguration umschaltbar ist; wobei das mindestens eine Musterelement eine erste geometrische Form umfasst, wenn sich das mindestens eine optische Element in der ersten Konfiguration befindet, und wobei das mindestens eine Musterelement eine zweite geometrische Form umfasst, wenn sich das mindestens eine optische Element in der zweiten Konfiguration befindet, wobei sich die erste geometrische Form von der zweiten geometrischen Form unterscheidet.

13. Vorrichtung (100) nach einem der Ansprüche 1 bis 12, wobei das mindestens eine optische Element mindestens ein diffraktives optisches Element, mindestens ein refraktives optisches Element und/oder mindestens eine Metaoberfläche zum Bereitstellen des Beleuchtungsmusters (630) umfasst, wobei vorzugsweise das mindestens eine optische Element eine Mikrolinse umfasst.

14. Nichttherapeutisches Verfahren zum Formen eines Beleuchtungsmusters (630) von Laserpulsen (410a, 410b) zur Gewebebehandlung, wobei das Verfahren die folgenden Schritte umfasst:
Bereitstellen mindestens eines Laserpulses (410a, 410b);
Formen eines Beleuchtungsmusters (630) des mindestens einen Laserpulses (410a, 410b) derart, dass das Beleuchtungsmuster (630) mindestens ein Musterelement (633, 634, 635) mit einer ersten Breite von weniger als 300 Mikrometern umfasst; und
wobei eine effektive Oberflächenabdeckung des Beleuchtungsmusters (630) mehr als 10 % beträgt;
a) wobei das Beleuchtungsmuster (630) ein Gitter umfasst, das das mindestens eine Musterelement umfasst, und wobei das Gitter ein Gitter aus dicht gepackten Ringen, wobei die benachbarten Ringe einander berühren, oder ein Gitter aus dicht gepackten abgerundeten sechseckigen Ringen, wobei die benachbarten abgerundeten sechseckigen Ringe einander berühren, umfasst, oder
b) wobei das Beleuchtungsmuster (630) ein Netz umfasst, das das mindestens eine Musterelement (633, 634, 635) umfasst; und
wobei das Netz ein Wabengitter umfasst.

## Revendications

1. Un appareil (100) pour le traitement par laser d'une surface tissulaire (110, 120), l'appareil (100) comprenant :
au moins un élément optique pour illuminer la surface tissulaire (110, 120) avec au moins une impulsion laser (410a, 410b) avec un motif d'illumination (610, 620, 630) ;
dans lequel l'au moins un élément optique est configuré de telle manière que :
le motif d'illumination (630) comprenne au moins un élément de motif (633, 634, 635) avec une première largeur inférieure à 300 micromètres ; et
une couverture efficace de surface du motif d'illumination (630) soit supérieure à 10 % ;
a) dans lequel le motif d'illumination (630) comprend un réseau comprenant l'au moins un élément de motif, et dans lequel le réseau comprend un réseau d'anneaux fortement tassés, les anneaux voisins se touchant l'un l'autre, ou un réseau d'anneaux hexagonaux arrondis fortement tassés, les anneaux hexagonaux arrondis voisins se touchant l'un l'autre ; ou
b) dans lequel le motif d'illumination (630) comprend un maillage comprenant l'au moins un élément de motif (633, 634, 635) ; et
dans lequel le maillage comprend un réseau en nid d'abeilles.

2. L'appareil (100) de la revendication 1, dans lequel une énergie d'impulsion de l'au moins une impulsion laser (410a, 410b) est comprise dans une plage telle que la fluence correspondante sur la surface tissulaire (110, 120) corresponde à un traitement non ablatif de la surface tissulaire (110, 120).

3. L'appareil (100) de la revendication 1 ou 2, dans lequel l'au moins une impulsion a une longueur d'impulsion plus courte que 2 ms, de préférence plus courte que 900 microsecondes.

4. L'appareil (100) de l'une des revendications 1 à 3, dans lequel l'appareil (100) comprend en outre une source laser, de préférence une source laser infrarouge, plus préférentiellement une source laser Er:YAG.

5. L'appareil (100) de l'une des revendications 1 à 4, dans lequel le tissu est prédéterminé et l'appareil (100) est configuré pour délivrer l'au moins une impulsion laser (410a, 410b) de telle manière que chacune des au moins une impulsion laser (410a, 410b) chauffe la surface tissulaire (110, 120) à une température supérieure à 60 °C.

6. L'appareil (100) de l'une des revendications 1 à 5, comprenant en outre :
des moyens pour recevoir une entrée utilisateur concernant la surface tissulaire (110, 120) à traiter ; et
des moyens pour adapter automatiquement au moins un paramètre de l'au moins une impulsion laser (410a, 410b) au moins en partie sur la base de l'entrée utilisateur.

7. L'appareil (100) de l'une des revendications 1 à 6, dans lequel au moins un élément de motif (130, 334, 635) présente une forme linéaire et/ou constitue au moins une partie d'une forme linéaire.

8. L'appareil (100) de l'une des revendications 1 à 7, dans lequel la première largeur correspond à une largeur de la forme linéaire et dans lequel la forme linéaire a une longueur (L) d'au moins 4 fois, de préférence au moins 10 fois, la première largeur.

9. L'appareil (100) de l'une des revendications 1 à 8, dans lequel le réseau comprend au moins un vecteur de réseau ayant une longueur (L) dans une plage allant de 0,1 mm à 10 mm, 0,5 mm à 5 mm, ou 1 mm à 3 mm.

10. L'appareil (100) de l'une des revendications 1 à 9, dans lequel la couverture efficace de surface du motif d'illumination (630) est inférieure à 50 %, inférieure à 40 %, ou inférieure à 30 %.

11. L'appareil (100) de l'une des revendications 1 à 10, dans lequel le motif d'illumination (630) formé des lignes de frontière d'un motif de pavage, de préférence d'un motif de pavage régulier.

12. L'appareil (100) de l'une des revendications 1 à 11, dans lequel l'au moins un élément optique peut basculer entre une première et une seconde configuration ;
dans lequel au moins un élément de motif présente une première forme géométrique lorsque l'au moins un élément optique est dans la première configuration et dans lequel l'au moins un élément de motif présente une seconde forme géométrique lorsque l'au moins un élément optique est dans la seconde configuration, la première forme géométrique étant différente de la seconde forme géométrique.

13. L'appareil (100) de l'une des revendications 1 à 12, dans lequel l'au moins un élément optique comprend au moins un élément optique diffractif, au moins un élément optique réfractif, et/ou au moins une métasurface pour produire le motif d'illumination (630), l'au moins un élément optique comprenant de préférence une microlentille.

14. Un procédé non thérapeutique pour la conformation d'un motif d'illumination (630) d'impulsions laser (410a, 410b) pour traiter un tissu, le procédé comprenant les étapes suivantes :
la production d'au moins une impulsion laser (410a, 410b) ;
la conformation d'un motif d'illumination (630) de l'au moins une impulsion laser (410a, 410b) de telle sorte que le motif d'illumination (630) comprenne au moins un élément de motif (633, 634, 635) avec une première largeur inférieure à 300 micromètres ; et
dans lequel une couverture efficace de surface du motif d'illumination (630) est supérieure à 10 % ;
a) dans lequel le motif d'illumination (630) comprend un réseau comprenant l'au moins un élément de motif, et dans lequel le réseau comprend un réseau d'anneaux fortement tassés, les anneaux voisins se touchant l'un l'autre, ou un réseau d'anneaux hexagonaux arrondis fortement tassés, les anneaux hexagonaux arrondis voisins se touchant l'un l'autre ; ou
b) dans lequel le motif d'illumination (630) comprend un maillage comprenant l'au moins un élément de motif (633, 634, 635) ; et
dans lequel le maillage comprend un réseau en nid d'abeilles.
